(19) Europäisches Patentamt
European Patent Office
Office européen des brevets

(11) Numéro de publication: **0 388 298 B1**

(12) # FASCICULE DE BREVET EUROPEEN

(45) Date de publication de fascicule du brevet: **21.06.95**

(51) Int. Cl.[6]: **C07D 401/04**, C07D 498/06, C07D 471/04, A61K 31/47, A61K 31/535, //(C07D498/06, 265:00,221:00),(C07D471/04, 221:00,221:00)

(21) Numéro de dépôt: **90400684.8**

(22) Date de dépôt: **14.03.90**

Le dossier contient des informations techniques présentées postérieurement au dépôt de la demande et ne figurant pas dans le présent fascicule.

(54) **Dérivés d'acides pyridone carboxyliques azétidinyl substitues, leur préparation et leur application en tant que médicaments.**

(30) Priorité: **16.03.89 FR 8903459**
**29.06.89 FR 8908695**
**20.11.89 FR 8915178**

(43) Date de publication de la demande:
**19.09.90 Bulletin 90/38**

(45) Mention de la délivrance du brevet:
**21.06.95 Bulletin 95/25**

(84) Etats contractants désignés:
**AT BE CH DE DK FR GB GR IT LI LU NL SE**

(56) Documents cités:
**EP-A- 0 047 005** **EP-A- 0 106 489**
**EP-A- 0 155 870** **EP-A- 0 241 206**
**EP-A- 0 314 362** **EP-A- 0 324 298**
**US-A- 3 998 808** **US-A- 4 183 923**

(73) Titulaire: **LABORATORIOS DEL DR. ESTEVE, S.A.**
**Av. Mare de Deu de Montserrat, 221**
**E-08026 Barcelona (ES)**

(72) Inventeur: **Pares Corominas, Juan**
**Padilla 349, 30 3a**
**E-08025 Barcelone (ES)**
Inventeur: **Colombo Pinol, Augusto**
**Av. Chile 36, 40 1a**
**E-08032 Barcelone (ES)**
Inventeur: **Frigola Constansa, Jordi**
**Av. Diagonal, 299 at.1a**
**E-08013 Barcelone (ES)**

(74) Mandataire: **Ahner, Francis et al**
**CABINET REGIMBEAU**
**26, avenue Kléber**
**F-75116 Paris (FR)**

EP 0 388 298 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

CHEM. PHARM. BULL., vol. 32, no. 12, 1984, pages 4907-4913; I. HAYAKAWA et al.: "Synthesis and antibacterial activities of substituted 7-oxo-2,3-dihydro-7H-pyrido[1,2,3-de][1,4] benzoxazine-6-carboxylic acids"

J. MED. CHEM., vol. 27, 1984, pages 1543-1548, American Chemical Society, US; H. EGAWA et al.: "Pyridonecarboxylic acids as antibacterial agents. 4. Synthesis and antibacterial activity of 7-(3-amino-1-pyrrolidinyl)-1-ethyl-6-fluoro-1,4-dihydro-4-oxo-1,8--naphthyridine-3-carboxylic acid and its analogues"

CHEMICAL SUBSTANCE INDEX, Furazan-Nin, vol. 106, janvier-juin 1987, Columbus, Ohio, US; "1,8-naphthyridine-3-carboxylic acid, 7-(3-amino-1-azetidinyl)-6-fluoro-1-(4-fluorophenyl(-1,4-dihydro-4-oxo-; 1,8-naphthyridine-3-carboxylic acid (Reg. nr. 105573-00-0), 6-fluoro-1-(4-fluorophenyl)-1,4-dihydro-4-oxo--7-[3-[(trifluoroacetyl)amino]-1-azetidinyl]-, ethyl ester (Reg. nr. 105587-75-5)", & CHEMICAL ABSTRACTS, vol. 106, 5 janvier 1987, page 470, résumé no. 4996j, Columbus, Ohio, US; & JP-A-61 137 885 (DAINIPPON PHARMACEUTICAL CO., LTD) 25-06-1986

CHEMICAL ABSTRACTS, 1982-1986, Eleventh Collective Index, Chemical Substances, vol. 96-105, Columbus, Ohio, US; "1,8-Naphthyridine-3-carboxylic acid, 1-cyclopropyl-6-fluoro-1,4-dihydro-7-(3-hydroxy-1-azetidinyl)-4-oxo-(Reg. nr. 99759-88-3); 1,8-naphthyridine-3-carboxylic acid, 1-cyclopropyl-6-fluoro-1,4-dihydro-7-(3-hydroxy-1-azetidinyl)-4-oxo-, ethyl ester (Reg. nr. 99759-89-4)", & CHEMICAL ABSTRACTS, vol. 104, no. 7, 17 février 1986, page 521, résumé no. 50861t, Columbus, Ohio, US; & JP-A-60 126 284 (DAINIPPON PHARMACEUTICAL CO., LTD) 05-07-1985 (Cat. D)

CHEMICAL ABSTRACTS, 1982-1986, Eleventh Collective Index, Chemical Substances, vol. 96-105, Columbus, Ohio, US; "1,8-naphthyridine-3-carboxylic acid, 1-cyclopropyl-6-fluoro-1,4-dihydro-4-oxo-7-[3[(trifluoroacetyl)amino]-1-azetidinyl]-, ethyl ester (Reg. nr. 98417-02-8); 1,8-naphthyridine-3-carboxylic acid, 7-(3-amino-1-azetidinyl)-1-cyclopropyl-6-fluoro-1,4-dihydro-4-oxo--(Reg. nr. 98417-03-9); 1,8-naphthyridine-3-carboxylic acid, 7-(3-amino-1-azetidinyl)-1-cyclopropyl-6-fluoro-1,4-dihydro-4-oxo, monohydrochioide (Reg. nr. 98417-04-0)", & CHEMICAL ABSTRACTS, vol. 103, no. 17, 28 octobre 1985, page 720, résumé no. 141933p, Columbus, Ohio, US; & JP-A-60 89 480 DAINIPPON PHARMACEUTICAL CO., LTD)

CHEMICAL ABSTRACTS, vol. 56, no. 9, 30 avril 1962, résumé no. 10069g-10071a, Columbus, Ohio, US; H. TANIYAMA et al.: "Nocardamin and its related compounds. I. Synthesis of 1-(RHO-TOSYL)-2-azetidineethanol", & YAKUGAKU ZASSHI 81, 1493-6(1961)

## Description

La présente invention se rapporte à des nouveaux dérivés azétidiniques des acides pyridonecarboxyliques 1,4-dihydro-4-oxoquinoléine-3-carboxylique, 1,8-naphtyridine-4-oxo-3-carboxylique et 2,3 dihydro-7-oxo-7H-pyrido[1,2,3-de]-1,4-benzoxazine-6- carboxylique, les sels thérapeutiquement acceptables de ces composés, leur procédé de préparation, ainsi que leur application en tant que médicaments.

Les composés objets de la présente invention peuvent être utilisés dans l'industrie pharmaceutique comme intermédiaires et pour la préparation de médicaments.

Dans les brevets Eur. Pat. Appl. EP. 106489 ; Eur. Pat. Appl. EP 153163 ; Japan Kokkai JP 58/72589 (83/72589) ; Japan Kokkai JP 60/89840 (85/89840) et Japan Kokkai JP 60/126284 (85/126284) sont décrites quelques azétidines 3-monosubstituées attachées à la position 7 de quelques quinolones et naphtyridines.

Dans la demande de brevet Français FR 87.18289 et son addition 88.09816 sont décrites quelques azétidines mono ou disubstituées dans leur position 3 et qui sont attachées à la position 7 de quelques quinolones et pyrido-benzoxazines.

Nous avons maintenant découvert que les nouveaux dérivés azétidiniques des acides 1,4-dihydro-4-oxoquinoléine-3-carboxylique, 1,8-naphtyridine-4-oxo-3-carboxylique et 2,3-dihydro-7-oxo-7H-pyrido[1,2,3-de]-1,4-benzoxazine-6-carboxylique, qui font l'objet de la présente invention, présentent une très bonne activité anti-microbienne.

Les composés objets de la présente invention répondent à la formule générale I.

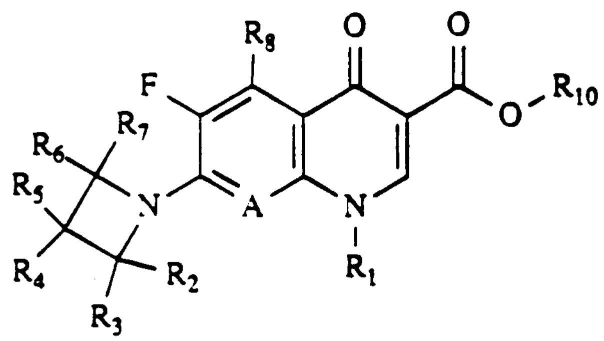

I

dans laquelle A représente un atome d'azote ou bien un atome de carbone avec un atome d'hydrogène attaché (C-H), ou bien un atome de carbone avec un halogène attaché (C-X), dans ce cas X représente un atome de chlore, de fluor ou de brome ou bien un atome de carbone avec un radical hydroxy (C-OH).

$R_1$ représente un radical alkyle ou cyclolkyle inférieur, un radical halogénoalkyle inférieur, un radical aryle ou un radical aryle substitué, notamment par un ou plusieurs atome(s) de fluor.

$R_2$ et $R_7$, égaux ou différents, représentent un atome d'hydrogène ou un radical alkyle inférieur.

$R_3$, $R_5$ et $R_6$, égaux ou différents, représentent un atome d'hydrogène, un radical alkyle inférieur, un radical aminoalkyle, un radical alkylamino, un radical alkylaminoalkyle.

$R_4$ représente un atome d'hydrogène, un radical alkyle inférieur, un radical hydroxyle, un radical amino, un radical aminoalkyle, un radical alkylamino, un radical dialkylamino, un radical hétérocyclique azoté de préférence aromatique pouvant être un cycle de trois à six maillons, un radical alkylaminoalkyle, un radical alkylcarboxamido, et dans ce dernier cas, le radical alkyle pouvant être substitué par un ou plusieurs halogènes, un radical arylsulfonyloxy, un radical alkylsulfonyloxy, un radical carboxamido, pouvant être substitué ou non sur l'azote, ou un radical cyano.

$R_8$ représente un atome d'hydrogène, un radical nitro, un radical amino ou amino substitué.

A et $R_1$ peuvent former ensemble une liaison représentée par un groupe C-CH2-CH2-CHR$_9$- ou C-O-CH2-CHR$_9$-dans lesquels $R_9$ représente un atome d'hydrogène ou un radical alkyle inférieur, et dans ce dernier cas, on a un centre chiral avec une configuration "R" ou "S".

$R_{10}$ représente un atome d'hydrogène ou un radical alkyl inférieur de C1 à C4.

Les substituants azétidiniques peuvent avoir, selon le nombre, la nature et la position relative des substituants, jusqu'à trois centre chiraux, chacun d'eux avec une configuration "R" ou "S", ainsi que leurs sels d'acides minéraux tels les chlorhydrates, ou d'acides organiques tels les toluènes sulfonates ou méthylsulfonates physiologiquement acceptables.

La stéréochimie des produits objet de la présente invention est déterminée par celle des produits de départ. Par sélection de la stéréoisométrie de chacun des produits de départ on peut obtenir tous les

stéréoisomères possibles et dans le cas où le produit de réaction est un mélange stéréoisomérique, les composants peuvent être séparés et leur configuration établie par des procédés bien connus.

Les nouveaux dérivés de formule générale I peuvent être préparés, conformément à l'invention, selon la méthode suivante :

Par réaction d'un composé de formule générale II

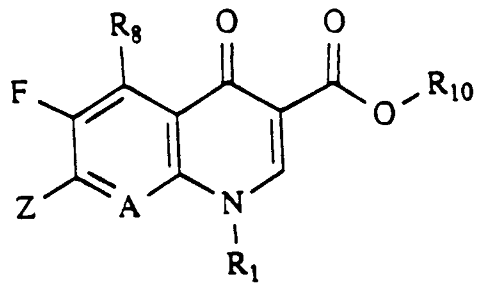

II

dans laquelle A, $R_1$, $R_8$ et $R_{10}$ ont les significations mentionnées précédemment et Z représente un atome d'halogène, de préférence un chlore ou un fluor, avec une azétidine, de formule générale III

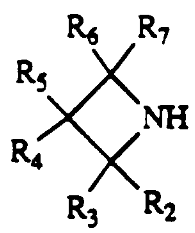

III

dans laquelle $R_2$, $R_3$, $R_4$, $R_5$, $R_6$ et $R_7$ ont les significations mentionnées précédemment.

Les composés hétérocycliques de formule générale II que l'on peut utiliser comme matières de départ pour préparer les composés de l'invention, sont des composés décrits, comme dans H. Koga, A. Itoh, S. Murayama, S. Suzue et T. Irikura J. Med. Chem., 1980. 23, 1358. ou bien dans H. Egawa, T. Miyamoto, A. Minamida, Y. Nishimura, H. Okada, H. Uno, J. Matsumoto, J. Med. Chem. 1984, 27, 1543.

D'autre part, les composés de formule générale III, qui constituent les autres matières de départ pour la préparation des composés de l'invention selon la formule générale I, sont connus ou bien sont synthétisés comme par exemple dans A.G. Anderson et R. Lok, J. Org. Chem. 1972, 37, 3953 ou bien dans R.H. Higgins et N.H. Cromwell, J. Heterocycl. Chem., 1971, 8, 1059 et aussi dans N.H. Cromwell et B. Phillips Chem. Rews., 1979, 79, 331.

Les azétidines de formule générale III peuvent avoir, selon le nombre, la nature et la position relative des substituants, jusqu'à trois centres chiraux, et on peut obtenir les différents stéréoisomères soit par synthèse assymétrique soit par différentes sortes de séparations, selon les procédés connus dans la chimie organique.

La réaction s'effectue en présence d'un solvant adéquat, par exemple le diméthylsulfoxyde, le diméthylformamide, la pyridine, les trialkylamines comme la triéthylamine, le chlorure de méthylène, le chloroforme , ou bien des éthers comme le tétrahydrofuranne ou le dioxanne, ou des mélanges de ces solvants.

Les températures les plus adéquates oscillent entre la température ambiante et la température de reflux du solvant, et le temps réactionnel est compris entre 1 heure et 24 heures.

Dans les exemples suivants on indique la préparation de nouveaux dérivés selon l'invention. On décrira également quelques formes d'emploi.

Les exemples ci-après, donnés à simple titre d'illustration ne doivent cependant, en aucune façon, limiter l'étendue de l'invention.

Exemple A. - Préparation de l'acide 1-cyclopropyl-6,8-difluoro-7-(3-méthyl-3-méthylamino-1-azétidinyl)-1,4-dihydro-4-oxo-3-quinoléincarboxylique.

On chauffe à reflux pendant 2 heures un mélange de 1,35 g (4,8 mmoles) de l'acide 1-cyclopropyl-6,7,8-trifluoro-1,4-dihydro-4-oxo-3-quinoléincarboxylique, 1,45 g (6,2 mmoles) de chlorhydrate de 3-méthyl-3-trifluoroacétamidoazétidine et 1 ml de triéthylamine dans 15 ml de pyridine. On évapore sous vide, on dilue avec de l'eau glacée, on filtre et on lave à l'eau. On obtient ainsi 2,2 g de l'acide 1-cyclopropyl-6,8-difluoro-7-(3-méthyl-3-trifluoroacétamido-N-méthyl-1-azétidinyl)-1,4-dihydro-4-oxo-3-quinoléincarboxylique, de point de fusion 291-4 °C, qui est ensuite hydrolysé en le chauffant dans un mélange de 4 ml de soude 10 % et 20 ml d'eau avec 1 ml d'éthanol, pendant 1 heure. On filtre à chaud, on acidifie avec de l'acide acétique, on filtre et on lave à l'eau. On obtient ainsi 1,57 g de l'acide 1-cyclopropyl-6,8-difluoro-7-(3-méthyl-3-méthylamino-1-azétidinyl)-1,4-dihydro-4-oxo-3-quinoléincarboxylique, de point de fusion > 300 °C.

Données spectroscopiques :

$^1$H RMN, $\delta$ ,J = Hz,[DMSO-TFAA]; 1,1(m,4H); 1,65(s, 3H); 2,7 (s, 3H); 4,0(m, 1H); 4,5(AB, J = 7, 4H); 7,75-(d,J = ,1H); 8,6(s, 1H); 9,4(élargie, 2H)

IR(KBr).-2918, 1731, 1622, 1470 $cm^{-1}$.

Exemple B. - Préparation de l'acide 1-cyclopropyl-6-fluoro-7-(3-diméthylamino-3-méthyl-1-azétidinyl)-1,4-dihydro-4-oxo-3-quinoléincarboxylique.

On chauffe à reflux pendant 2 heures un mélange de 1,32 g (5 mmoles) de l'acide 1-cyclopropyl-6,7-difluoro-1,4-dihydro-4-oxo-3-quinoléincarboxylique, 1,31 g (7 mmoles) de l'hydrochlorure de 3-méthyl-3-diméthylaminoazétidine et 3 ml de triéthylamine dans 10 ml de pyridine. On évapore, on laisse refroidir, on additionne de l'eau glacée, on filtre et on lave à l'eau, l'éthanol et l'éther, et on obtient ainsi 1,8 g de l'acide 1-cyclopropyl-6-fluoro-7-(3-diméthylamino-3-méthyl-1-azétidinyl) -1,4-dihydro-4-oxo-3-quinoléincarboxylique, de point de fusion 298-301 °C.

Données spectroscopiques :

$^1$H RMN, $\delta$ ,J = Hz,[DMSO-TFAA]; 1,16(m,4H); 1,67(s, 3H); 2,78 (s, 6H); 3,67(m, 1H); 4,29(AB,J = 20, J = 9,3, 4H); 7,0(d, J = 7,5 1H); 7,85(d, J = 12,9, 1H)); 8,6(s, 1H);

IR(KBr).-1712, 1629, 1521, 1476 $cm^{-1}$.

Exemple 1 . - Préparation de l'acide 1-cyclopropyl-6-fluoro-7-(3-amino-3-méthyl-1-azétidinyl)-1,4-dihydro-4-oxo-1,8-naphtyridine-3-carboxylique.

On chauffe à reflux pendant 3 heures un mélange de 0,5 g (1,8 mmoles) de l'acide 1-cyclopropyl-6-fluoro-7-chloro-1,4-dihydro-4-oxo-1,8-naphtyridine-3-carboxylique, 0,34 g (2,1 mmoles) d'hydrochlorure de 3-méthyl-3-aminoazétidine et 0,5 ml de triéthylamine dans 10 ml de pyridine. On laisse refroidir, on filtre et on lave à l'eau. On obtient ainsi 0,52 g de l'acide 1-cyclopropyl-6-fluoro-7-(3-amino-3-méthyl-1-azétidinyl)-1,4-dihydro-4-oxo-1,8-naphtyridine-3-carboxylique de point de fusion 285-7 °C.

Données spectroscopiques :

$^1$H RMN, $\delta$ ,J = Hz,[DMSO-TFAA]; 8,59(s,1H); 8,4 (élargie, 2H); 8,0 (d, J = 13, 1H); 4,4(AB, J = 7, 4H); 3,65(m, 1H); 1,65 (s, 3H); 1,1(m, 4H)

IR(KBr).- 2943, 1629, 1447 $cm^{-1}$.

Exemple 2 . - Préparation de l'acide 1-cyclopropyl-6-fluoro-7-(<u>trans</u>-3-amino-2-méthyl-1-azétidinyl)-1,4-dihydro-4-oxo-1,8-naphtyridine-3-carboxylique.

Par une procédure tout à fait semblable à celle décrite dans l'exemple 1 , on obtient l'acide 1-cyclopropyl-6-fluoro-7-(<u>trans</u>-3-amino-2-méthyl-1-azétidinyl)-1,4-dihydro-4-oxo-1,8-naphtyridine-3-carboxylique de point de fusion 211-8 °C.

Données spectroscopiques :

$^1$H RMN, $\delta$, J = Hz,[DMSO-TFAA] ; 8,6 (s,1H) ; 8,4 (élargie, 2H) ; 7,95 (d, J = 13, 1H) ; 4,7 (m, 2H) ; 4,25 (m, 1H) ; 3,6 (m, 2H) ; 1,55 (d, J = 6, 3H) ; 1,1 (m, 4H).

IR (KBr).-2943, 1629, 1447 $cm^{-1}$.

Exemple 3. - Préparation de l'acide 1-cyclopropyl-6,8-difluoro-7-(trans-3-diméthylamino-2-méthyl-1-azétidinyl)-1,4-dihydro-4-oxo-3-quinoléincarboxylique.

Par une procédure tout à fait semblable à celle décrite dans l'exemple B, on obtient l'acide 1-cyclopropyl-6,8-difluoro-7-(trans-3-diméthylamino-2-méthyl-1-azetidinyl)-1,4-dihydro-4-oxo-3-quinoléincarboxylique, de point de fusion 149-151 ° C.

Données spectroscopiques :

$^1$H RMN, $\delta$ ,J = Hz,[DMSO-TFAA]; 8,61(s, 1H); 7,75(dd, J = 13, J = 1,5, 1H); 4,98(m, 1H); 4,67(m, 1H); 4,34 (m, 1H); 3,92(m, 2H); 2,83 (s, 6H); 154 (d, J = 6,3H); 1,16 (d, J = 6, 4H).

IR(KBr).-1729, 1627, 1523, 1459, 1328 $cm^{-1}$.

Exemple 4. - Préparation de l'acide 1-cyclopropyl-6,8-difluoro-7-(trans-3-méthylamino-2-méthyl-1-azétidinyl)-1,4-dihydro-4-oxo-3-quinoléincarboxylique.

Par une procédure tout à fait semblable à celle de l'exemple A, on obtient l'acide 1-cyclopropyl-6,8-difluoro-7-(trans-3-méthylamino-2-méthyl-1-azétidinyl)-1,4-dihydro-4-oxo-3-quinoléincarboxylique de point de fusion 241-6 ° C.

Données spectroscopiques :

$^1$H-RMN, $\delta$ ,J = Hz[DMSO-TFA]; 9,23 (e,2H); 8,65 (s,1H); 7,77 (d, $^1$H,J = 13Hz); 4,87 (m,2H); 3,77 (m,1H); 2,66 (s,3H); 1,58 (d,3H); 1,58 (d,3H,J = 5Hz); 1,19 (d,4H,J = 5,6Hz).

IR(KBr).- 2930, 1625, 1461, 1322 $cm^{-1}$.

Exemple 5. - Préparation de l'acide 1-cyclopropyl-6,8-difluoro-7-(cis-3-amino-2-méthyl-1-azétidinyl)-1,4-dihydro-4-oxo-3-quinoléincarboxylique.

Par une procédure tout à fait semblable à celle décrite dans l'exemple B, on obtient l'acide 1-cyclopropyl-6,8-difluoro -7-(cis-3-amino-2-méthyl-1-azétidinyl)-1,4-dihydro-4-oxo-3-quinoléincarboxylique de point de fusion 215-8 ° C.

Données spectroscopiques :

$^1$H-RMN, $\delta$ ,J = Hz[DMSO-TFA]; 8,57 (s,1H); 8,39 (e,2H); 7,69 (d,J = 13,1H); 5,01 (m,1H); 4,39 (m,3H); 3,99 (m,1H); 1,48 (d,$^J$ = 6,3H); 1,12 (d,J = 6,4H).

IR(KBr).- 3385, 1725, 1626, 1523, 1412, 1337, 803 $cm^{-1}$.

Exemple 6. - Préparation de l'acide 1-cyclopropyl-6,8-difluoro-7-(r-3-amino-3-trans-2-diméthyl-1-azétidinyl)-1,4-dihydro-4-oxo-3-quinoléincarboxylique.

Par une procédure tout à fait semblable à celle décrite dans l'exemple B, on obtient l'acide 1-cyclopropyl-6,8-difluoro-7-(r-3-amino-3-trans-2-dimethyl-1-azétidinyl)-1,4-dihydro-4-oxo-3-quinoléincarboxylique de point de fusion 265-268 ° C.

Données spectroscopiques :

$^1$H-RMN, $\delta$ ,J = Hz[DMSO-TFA]; 8,63 (s,1H); 7,77 (d,J = 13,1H); 4,83 (m,1H); 4,33 (m,2H); 4,05 (m,1H); 1,49 (s,3H); 1,44 (d,$^J$ = 6,3H); 1,17 (d,J = 6,4H).

IR(KBr).- 3380, 1719, 1628, 1460 $cm^{-1}$.

Exemple 7. - Préparation de l'acide 5-amino-1-cyclopropyl-6,8-difluoro-7-(trans-3-amino-2-methyl-1-azétidinyl)-1,4-dihydro-4-oxo-3-quinoléincarboxylique.

Par une procédure tout à fait semblable à celle de l'exemple B, on obtient l'acide 5-amino-1-cyclopropyl-6,8-difluoro-7-(trans-3-amino-2-méthyl-1-azétidinyl)-1,4-dihydro-4-oxo-3-quinoléincarboxylique de point de fusion 206-210 ° C.

Données spectroscopiques :

$^1$H-RMN, $\delta$, [OMSO-TFA]; 1,05 (m,4H); 1,40 (d,J = 5Hz,3H); 3,46 (m,1H); 3,78 (m,1H); 4,0 (m,1H); 4,59 (m,2H); 8,25 (e,2H); 8,33 (s,1H).

IR(KBr).- 3419, 1710, 1632, 1518, 1432, 1304 $cm^{-1}$.

Exemple 8. - Préparation de l'acide 1-cyclopropyl-6,8-difluoro-7-(cis-3-amino-2-éthyl-1-azétidinyl)-1,4-dihydro-4-oxo-3-quinoléincarboxylique.

Par une procédure tout à fait semblable à celle de l'exemple B, on obtient l'acide 1-cyclopropyl-6,8-difluoro -7-(cis-3-amino-2-éthyl-1-azétidinyl)-1,4-dihydro-4-oxo-3-quinoléincarboxylique de point de fusion 230-234 ° C (dec.).

Données spectroscopiques :

$^1$H-RMN, $\delta$ ,J = Hz[DMSO-TFA]; 0,94 (t,J = 6,5Hz,3H); 1,17 (m,4H); 1,92 (m,2H); 4,09 (m,1H); 4,35 (m,3H); 4,82 (m,1H); 7,74 (d,$^J$ = 13,3Hz,1H); 8,49 (m,2H), 8,60 (s,1H).

IR(KBr).- 3393, 3318, 1726, 1628, 1544, 1498, 1491, 806 $cm^{-1}$.

Exemple 9. - Préparation de l'acide 1-(2,4-difluorophényl)-6,8-difluoro-7-(trans-3-amino-2-méthyl-1-azétidinyl)-1,4-dihydro-4-oxo-3-quinoléincarboxylique.

Par une procédure tout à fait semblable à celle décrite dans l'exemple B, on obtient l'acide 1-2,4-difluorophényl)-6,8-difluoro-7-(trans-3-amino-2-méthyl-1-azétidinyl)-1,4-dihydro-4-oxo-3-quinoléincarboxylique de point de fusion 200-204 ° C.

Données spectroscopiques :

$^1$H-RMN, $\delta$ ,J = Hz[DMSO-TFA]; 1,4 (d,J = 6Hz,3H); 3,65 (m,1H); 4,1 (m,1H); 4,6 (m,2H); 7,81 (m,4H); 8,34 (é,2H); 8,61 (s,1H).

IR(KBr).- 1619, 1509, 1474 $cm^{-1}$.

Exemple 10. - Préparation de l'acide 1-(2,4-difluorophényl)-6-fluoro-7-(trans-3-amino-2-méthyl-1-azétidinyl)-1,4-dihydro-4-oxo-3-quinoléincarboxylique.

Par une procédure tout à fait semblable à celle décrite dans l'exemple B, on obtient l'acide 1-2,4-difluorophényl)-6-fluoro-7-(trans-3-amino-2-méthyl-1-azétidinyl)-1,4-di-hydro-4-oxo-3-quinoléincarboxylique de point de fusion 203-205 ° C.

Données spectroscopiques :

$^1$H-RMN, $\delta$ ,J = Hz[DMSO-TFA]; 1,32 (d,J = 6Hz,3H); 3,78 (m,2H); 4,3 (m,2H); 5,78 (d,J = 7Hz,1H); 8,0 (m,4H); 8,3 (é,2H); 8,7 (s,1H).

IR(KBr).- 2950, 1628, 1509 $cm^{-1}$.

Exemple 11. - Préparation de l'acide 1-(4-fluorophényl)-6-fluoro-7-(trans-3-amino-2-méthyl-1-azétidinyl)-1,4-dihydro-4-oxo-3-quinoléincarboxylique.

Par une procédure tout à fait semblable à celle décrite dans l'exemple B, on obtient l'acide 1-(4-fluorophényl)-6-fluoro-7-(trans-3-amino-2-méthyl-1-azétidinyl)-1,4-di hydro-4-oxo-3-quinoléincarboxylique de point de fusion 235-239 ° C.

Données spectroscopiques :

$^1$H-RMN, $\delta$ ,J = Hz[DMSO-TFA]; 8,64 (s,1H); 8,25 (é,2H); 8,1 (d,J = 13Hz,1H); 7,75 (m,4H); 5,84 (d,J = 8Hz,1H); 4,25 (m,2H); 3,81 (m,2H); 1,31 (d,J = 6Hz,3H).

IR(KBr).- 3388, 1724, 1630, 1505 $cm^{-1}$.

Exemple 12. - Préparation de l'acide 1-(2-fluoroéthyl)-6,8-difluoro-7-(trans-3-amino-2-méthyl-1-azétidinyl)-1,4-dihydro-4-oxo-3-quinoléincarboxylique.

Par une procédure tout à fait semblable à celle décrite dans l'exemple B, on obtient l'acide 1-(2-fluoroéthyl)-6,8-difluoro-7-(trans-3-amino-2-méthyl-1-azétidinyl)-1,4-di hydro-4-oxo-3-quinoléincarboxylique de point de fusion 222-224 ° C.

Données spectroscopiques :

$^1$H-RMN, $\delta$ ,J = Hz[DMSO-TFA]; 1,53 (d,J = 6Hz,3H); 3,7 (m,1H); 4,27 (m,2H); 4,7 (m,3H); 5,0 (m,2H); 7,9 (d,J = 12Hz,1H); 8,44 (é,2H), 8,8 (s,1H).

IR(KBr).- 2985, 1632, 1476 $cm^{-1}$.

Exemple 13. - Préparation de l'acide 1-(2-fluoroéthyl)-6-fluoro-7-(trans-3-amino-2-méthyl-1-azétidinyl)-1,4-dihydro-4-oxo-3-quinoléincarboxylique.

Par une procédure tout à fait semblable à celle décrite dans l'exemple B, on obtient l'acide 1-(2-fluoroéthyl)-6-fluoro-7-(trans-3-amino-2-méthyl-1-azétidinyl)-1,4-dihydro-4-oxo-3-quinoléincarboxylique de point de fusion 205-220 ° C.

Données spectroscopiques :

$^1$H-RMN, δ ,J = Hz[DMSO-TFA]; 1,52 (d,J = 6Hz,3H); 3,92 (m,$^2$H); 4,6 (m,4H); 5,0 (m,2H); 6,75 (d,J = 7Hz,1H); 7,9 (d,J = 13Hz, 1H); 8,4 (é,2H), 8,83 (s,1H).

IR(KBr).- 3100, 1631, 1490, 1341 $cm^{-1}$.

Exemple 14. - Préparation de l'acide 1-(4-fluorophényl)-6,8-difluoro-7-(trans-3-amino-2-méthyl-1-azétidinyl)-1,4-dihydro-4-oxo-3-quinoléincarboxylique.

Par une procédure tout à fait semblable à celle décrite dans l'exemple B, on obtient l'acide 1-(4-fluorophényl)-6,8-difluoro-7-(trans-3-amino-2-méthyl-1-azétidinyl)-1,4-dihydro-4-oxo-3-quinoléincarboxylique de point de fusion 223-229 ° C.

Données spectroscopiques :

$^1$H-RMN, δ ,J = Hz[DMSO-TFA]; 8,45 (s,1H); 8,3 (é,2H); 7,8 (m,5H); 4,55 (m,2 H); 4,02 (m,1H); 3,64 (m,1H); 1,4 (d, J = 6Hz, 3H).

IR(KBr).- 3420, 1623, 1578, 1472 $cm^{-1}$.

Exemple 15. Préparation de l'acide (-)-(3S)-10 -[3-(S)-amino-2-(R)-méthyl-1-azétidinyl]-9-fluoro-2,3-dihydro-3-méthyl-7-oxo-7H-pyrido[1,2,3-de][1,4]benzoxazine-6-carboxylique.

Par une procédure tout à fait semblable à celle de l'exemple B, on obtient l'acide (-)-(3S)-10-[3-(S)-amino-2-(R)-méthyl-1-azétidinyl]-9-fluoro-2,3-dihydro-3-méthyl-7-oxo-7H-pyrido[1,2,3-de][1,4]benzoxazine-6-carboxylique de point de fusion 217-219 ° C.

$[\alpha]_D^{20}$ = -106,8 (c = 0,31, NaOH 0,5N)

Données spectroscopiques :

$^1$H-RMN, δ ,J = Hz,[DMSO-TFA]; 1,50 (m,6H); 3,7 (m,1H); 4,00-5,10 (m,6H); 7,58 (d,J = 14,0Hz,1H); 8,35 (é,3H); 8,92 (s,1H).

IR(KBr).- 3425, 2975, 1623, 1472, 1333 $cm^{-1}$.

Exemple 16. Préparation de l'acide ( + )-(3R)-10 -[3-(R)-amino-2-(S)-méthyl-1-azétidinyl]-9-fluoro-2,3-dihydro-3-méthyl-7-oxo-7H-pyrido[1,2,3-de][1,4]benzoxazine-6-carboxylique.

Par une procédure tout à fait semblable à celle décrite dans l'exemple B, on obtient l'acide ( + )-(3R)-10-[3-(R)- amino-2-(S)-méthyl-1-azétidinyl]-9-fluoro-2,3-dihydro-3-méthyl-7-oxo-7H-pyrido[1,2,3-de][1,4]-benzoxazine -6-carbo xylique de point de fusion 215-217 ° C.

$[\alpha]_D^{20}$ = + 104,7 (c = 0,25, NaOH 0,5N)

Données spectroscopiques :

$^1$H-RMN, δ ,J = Hz,[DMSO-TFA]; 1,50 (m,6H); 3,7 (m,1H); 4,00-5,10 (m,6H); 7,58 (d,J = 14,0Hz,1H); 8,35 (é,3H); 8,92 (s,1H).

IR(KBr).- 3425, 2975, 1623, 1472, 1333 $cm^{-1}$.

Exemple 17.- Préparation de l'acide ( + )-1-cyclopropyl-6,8-difluoro-7-(3-(S)-amino-2-(R)-methyl-1-azétidinyl)-1,4-dihydro-4-oxo-3-quinoléincarboxylique.

Par une procédure tout à fait semblable à celle décrite dansl'exemple B, on obtient l'acide ( + )-1-cyclopropyl-6,8-difluoro-7-(3-(S)-amino-2-(R)-methyl-1-azétidinyl)-1,4-dihydro-4-oxo-3-quinoléincarboxylique de point de fusion 229-231 ° C.

$[\alpha]_D^{20}$ = + 9,4 (c = 0,26, NaOH 0,5N)

Données spectroscopiques :

$^1$H-RMN, δ ,J = Hz,[DMSO-TFA]; 8,61 (s,1H); 8,32 (é,2H); 7.70 (dd,J = 13,J = 1,5,1H); 4,76 (m,2H); 4,09 (m,2H) ; 3,72 (m,1H); 1,53 (d,J = 6,3H); 1,16 (d,J = 6,4H).

IR(KBr).- 1719, 1630, 1578, 1466, 1402, 1319 $cm^{-1}$.

Exemple 18.- Préparation de l'acide (-)-1-cyclopropyl-6,8-difluoro-7-(3-(R)-amino-2-(S)-méthyl-1- azétidinyl)-1,4-dihydro-4-oxo-3-quinoléincarboxylique.

Par une procédure tout à fait semblable à celle décrite dansl'exemple B, on obtient l'acide (-)-1-cyclopropyl-6,8-difluoro-7(3-(R)-amino-2-(S)-méthyl-1-azétidinyl)-1,4-dihydro-4-oxo-3-quinoléincarboxylique de point de fusion 231-233 °C.

$[\alpha]_D^{20}$ = -10,6 (c = 0,27, NaOH 0,5N)

Données spectroscopiques :

$^1$H-RMN, $\delta$ ,J = Hz,[DMSO-TFA]; 8,61 (s,1H); 8,32 (é,2H); 7,70 (dd,J = 13,J = 1,5,1H); 4,76 (m,2H); 4,09 (m,2H); 3,72 (m, 1H); 1,53 (d,J = 6,3H); 1,16 (d,J = 6,4H).

IR(KBr).- 1719, 1630, 1578, 1466, 1402, 1319 $cm^{-1}$.

Exemple 19.- Préparation de l'acide (-)-1-cyclo -propyl-6-fluoro-7-(3-(S)-amino-2-(R)-méthyl-1-azétidinyl)-1,4-dihydro-4-oxo-1,8-naphtyridine-3-carboxylique.

Par une procédure tout à fait semblable à celle décrite dansl'exemple B, on obtient l'acide (-)-1-cyclopropyl-6-fluoro-7-(3-(S)-amino-2-(R)-méthyl-1-azétidinyl)-1,4-dihydro-4-oxo-1,8-naphtyridine-3-carboxylique de point de fusion 236-239 °C.

$[\alpha]_D^{20}$ = - 7,0 (c = 0,37, NaOH 0,5N)

Données spectroscopiques :

$^1$H-RMN, $\delta$ ,J = Hz,[DMSO-TFA]; 8,64 (s,1H); 8,35 (é,2H); 8 (d,J = 13Hz,1H); 4,7 (m,2H); 4,25 (m,1H); 3,65 (m,2H); 1,62 (d,J = 6Hz,3H); 1,1 (m,4H).

IR(KBr).- 2943, 1629, 1447 $cm^{-1}$.

Exemple 20.- Préparation de l'acide ( + )-1-cyclopropyl-6-fluoro-7-(3-(R)-amino-2-(S)-méthyl-1-azétidinyl)-1,4-dihydro-4-oxo-1,8-naphtyridine-3-carboxylique.

Par une procédure tout à fait semblable à celle décrite dansl'exemple B, on obtient l'acide ( + )-1-cyclopropyl-6-fluoro-7-(3-(R)-amino-2-(S)-méthyl-1-azétidinyl)-1,4-dihydro-4-oxo-1,8-naphtyridine-3-carboxylique de point de fusion 236-239 °C.

$[\alpha]_D^{20}$ = + 7,6 (c = 0,42, NaOH 0,5N)

Données spectroscopiques :

$^1$H-RMN, $\delta$ ,J = Hz,[DMSO-TFA]; 8,64 (s,1H); 8,35 (é,2H); 8 (d,J = 13Hz,1H); 4,7 (m,2H); 4,25 (m,1H); 3,65 (m, 2H); 1,62 (d,J = 6Hz,3H); 1,1 (M,4H).

IR(KBr).- 2943, 1629, 1447 $cm^{-1}$.

Exemple 21.- Préparation de l'acide ( + )-1-cyclo -propyl-6-fluoro-7-(3-(S)-amino-2-(R)-méthyl-1-azétidinyl)-1,4-dihydro-4-oxo-3-quinoléincarboxylique.

Par une procédure tout à fait semblable à celle décrite dansl'exemple B, on obtient l'acide ( + )-1-cyclopropyl-6-fluoro-7-(3-(S)-amino-2-(R)-méthyl-1-azétidinyl)-1,4-dihydro-4-oxo-3-quinoléincarboxylique de point de fusion 242-244 °C.

$[\alpha]_D^{20}$ = + 13,7 (c = 0,38, NaOH 0,5N)

Données spectroscopiques :

$^1$H-RMN, $\delta$ ,J = Hz,[DMSO-TFA]; 8,61 (s,1H); 8,37 (é,2H); 7,86 (d,J = 13,1H); 7,04 (d,J = 8,1H); 4,53 (m,2H); 3,92 (m,3H); 1,54 (d,J = 6,3H); 1,19 (d,J = 8,4H).

IR(KBr).- 1719, 1629, 1479, 1325 $cm^{-1}$.

Exemple 22.- Préparation de l'acide (-)-1-cyclo -propyl-6-fluoro-7-(3-(R)-amino-2-(S)-méthyl-1-azétidinyl)-1,4-dihydro-4-oxo-3-quinoléincarboxylique.

Par une procédure tout à fait semblable à celle décrite dans l'exemple B, on obtient l'acide (-)-1-cyclopropyl-6-fluoro-7-(3-(R)-amino-2-(S)-méthyl-1-azétidinyl)-1,4-dihydro-4-oxo-3-quinoléincarboxylique de point de fusion 242-244 °C.

$[\alpha]_D^{20}$ = -13,3 (c = 0,31, NaOH 0,5N)

Données spectroscopiques :

$^1$H-RMN, $\delta$ ,J = Hz,[DMSO-TFA]; 8,61 (s,1H); 8,37 (é,2H); 7,86 (d,J = 13,1H); 7,04 (d,J = 8,1H); 4,53 (m,2H); 3,92 (m,3H); 1,54 (d,J = 6,3H); 1,19 (d,J = 8,4H).

IR(KBr).- 1719, 1629, 1479, 1325 $cm^{-1}$.

Exemple 23. Préparation de l'acide (-)-(3S)-10 -[3-(R)-amino-2-(S)-méthyl-1-azétidinyl]-9-fluoro-2,3-dihydro-3-méthyl-7-oxo-7H-pyrido[1,2,3-de][1,4]benzoxazine-6-carboxylique de point de fusion 217-221 ° C.
$[\alpha]_D^{20}$ = -30,27 (c = 0,36, NaOH 0,5N)
Données spectroscopiques :
$^1$H-RMN, $\delta$ ,J = Hz,[DMSO-TFA]; 1,45 (d,J = 7,0Hz,3H); 1,52 (d, J = 6,0Hz,3H); 3,66 (m,1H); 4,00-5,00 (m,6H); 7,57 (d, J = 13,0Hz,1H); 8,36 (é,3H); 8,92 (s,1H).
IR(KBr).- 3393,2962,1718,1624,1529,1474,1131,800 $cm^{-1}$.

Exemple 24. Préparation de l'acide ( + )-(3R)-10 -[3-(S)-amino-2-(R)-méthyl-1-azétidinyl]-9-fluoro-2,3-dihydro-3-méthyl-7-oxo-7H-pyrido[1,2,3-de][1,4]benzoxazine-6-carboxylique.

Par une procédure tout à fait semblable à celle de l'exemple B, on obtient l'acide ( + )-(3R)-10-[3-(S)-amino-2-(R)-méthyl-1-azétidinyl]-9-fluoro-2,3-dihydro-3-méthyl-7-oxo-7H-pyrido[1,2,3-de][1,4]benzoxazine-6-carboxylique de point de fusion 217-219 ° C.
$[\alpha]_D^{20}$ = + 30,60 (c = 0,31, NaOH 0,5N)
Données spectroscopiques :
$^1$H-RMN, $\delta$ ,J = Hz,[DMSO-TFA]; 1,45 (d,J = 7,0Hz,3H); 1,52 (d, J = 6,0Hz,3H); 3,66 (m,1H); 4,00-5,00 (m,6H); 7,57 (d, J = 13,0Hz,1H); 8,36 (é,3H); 8,92 (s,1H).
IR(KBr).- 3393, 2962, 1718, 1624, 1529, 1474, 1131, 800 $cm^{-1}$.

Exemple 25. - Préparation de l'acide 1-cyclopropyl-6-fluoro-7-(3-méthyl-3-méthylamino-1-azétidinyl)-1,4-dihydro-4-oxo-1,8-naphtyridine-3-carboxylique.

Par une procédure tout à fait semblable à celle décrite dans l'exemple A, on obtient l'acide 1-naphtyridine-6-fluoro-7-(3-méthyl-3-trifluoro-méthyl-acétamidomethyl-1-azétidinyl)-1,4-dihydro-4-oxo-1,8-naphtyridine-3-carboxylique qui est ensuite hydrolysé pour conduire à l'acide 1-cyclopropyl-6-fluoro-7-(3-méthyl-3-méthylamino-1-azétidinyl)-1,4-dihydro-4-oxo-1,8-naphtyridine-3-carboxylique de point de fusion 283-286 ° C.
Données spectroscopiques :
$^1$H-RMN, $\delta$ ,J = Hz,[DMSO-TFA]; 1,0 (m,4H); 1,62 (s,3H); 2,62 (s,3H); 3,73 (m,1H); 4,38 (AB,J = 7,5,4H); 8 (d,J = 11,5Hz, 1H); 8,54 (s,1H); 9,34 (é,2H).
IR(KBr).- 2900, 1639, 1458 $cm^{-1}$.

Exemple 26. - Préparation du sel méthylsulfonate de l'acide 6-fluoro-7-(trans-2-méthyl-3-amino-1-azétidinyl)-1-cyclopropyl-1,4-dihydro-4-oxo-1,8-naphtyridine-3-carboxylique.

A une suspension de 0,6 g de l'acide 6-fluoro-7-(trans-2-méthyl-3-amino-1-azétidinyl)-1-cyclopropyl-1,4-dihydro-4-oxo-1,8-naphtyridine-3-carboxylique dans 20 ml d'éthanol à ébullition, on ajoute une solution de l'acide méthansulfonique dans de l'éthanol jusqu'à un pH légèrement acide (6). Après refroidissement, on filtre le solide précipité, on lave avec de l'éthanol froid et on obtient 0,55 g du sel méthylsulfonate de l'acide 6-fluoro-7-(trans-2-méthyl-3-amino-1-azétidinyl)-1-cyclopropyl-1,4-dihydro-4-oxo-1,8-naphtyridine-3-carboxylique de point de fusion 254-257 ° C.
Données spectroscopiques :
$^1$H-RMN, $\delta$ ,J = Hz,[DMSO-$d_6$]; 1,14 (m,4H); 1,63 (d,J = 6Hz,3H); 2,3 (s,3H); 3,5 (m,2H); 4,33 (m,1H); 4,64 (m,1H); 8,06 (d,J = 12Hz,1H); 8,37 (é,2H), 8,6 (s,1H).
IR(KBr).- 1710, 1648, 1462, 1232, 1162 $cm^{-1}$.

Exemple 27. - Préparation de l'acide (±)-1-(2,4-difluorophényl)-6-fluoro-7-(trans-2-méthyl-3-amino-1-azétidinyl)-1,4-dihydro-4-oxo-1,8-naphtyridine-3-carboxylique.

Par une procédure semblable à celle de l'exemple B on obtient l'acide (±)-1-(2,4-difluorophényl)-6-fluoro-7-(trans-2-méthyl-3-amino-1-azétidinyl)-1,4-dihydro-4-oxo-1,8-naphtyridine-3-carboxylique de point de fusion 220 ° C.
Données spectroscopiques :
$^1$H-RMN, $\delta$ ,J = Hz,[DMSO-TFA]; 1,25 (d,J = 6Hz,3H); 3,72 (m, 1H); 4,25 (m,3H); 7,15-7,85 (a.c., 3H); 8,14 (d,J = 11Hz, 1H); 8,25 (é,2H); 8,83 (s,1H).

IR(KBr).- 2925, 1632, 1513, 1451 $cm^{-1}$.

Exemple 28. - Préparation de l'acide (±)-8-chloro-1-cyclopropyl-6-fluoro-7-(trans-2-méthyl-3-amino-1-azétidinyl)-1,4-dihydro-4-oxo-3-quinoléincarboxylique.

Par une procédure tout à fait semblable à celle décrite dans l'exemple B, on obtient l'acide (±)-8-chloro -1-cyclopropyl-6-fluoro-7-(trans-2-méthyl-3-amino-1-azétidinyl)-1,4-dihydro-4-oxo-3-quinoléincarboxylique de point de fusion 226-230 °C.

Données spectroscopiques :

$^{1}$H-RMN, δ ,J = Hz,[DMSO-$d_6$-TFA]; 1,11 (m,4H); 1,54 (d,J = 6Hz, 3H); 3,7 (m,1H); 4,25 (m,2H); 5 (d,J = 14Hz,1H); 8,45 (é,2H); 8,73 (s,1H).

IR(KBr).- 2969, 1625, 1455, 1447 $cm^{-1}$.

Exemple 29. - Préparation de l'acide 8-chloro-1-cyclopropyl-6-fluoro-7-(3-méthyl-3-amino-1-azétidinyl) -1,4-dihydro-4-oxo-3-quinoléincarboxylique.

Par une procédure tout à fait semblable à celle décrite dans l'exemple B, on obtient l'acide 8-chloro-1-cyclopropyl-6-fluoro-7-(3-méthyl-3-amino-1-azétidinyl)-1,4-dihydro-4-oxo-3-quinoléincarboxylique, de point de fusion 284-285 °C.

Données spectroscopiques :

$^{1}$H-RMN, δ ,J = Hz,[DMSO-$d_6$-TFA]; 1,05 (m,4H); 1,57 (s,3H); 4,25 (m,1H); 4,51 (m,4H); 7,7 (d,J = 14Hz,1H); 8,43 (é,2H), 8,70 (s,1H).

IR(KBr).- 2945, 1639, 1611, 1444, 1356 $cm^{-1}$.

Exemple 30. - Préparation de l'acide (±)-8-chloro-1-(2,4-difluorophényl)-6-fluoro-7-(trans-2-méthyl-3-amino-1-azétidinyl)-1,4-dihydro-4-oxo-3-quinoléincarboxylique.

Par une procédure tout à fait semblable à celle décrite dans l'exemple B, on obtient l'acide (±)-8-chloro -1-(2,4-difluorophényl)-6-fluoro-7-(trans-2-méthyl-3-amino-1-azétidinyl)-1,4-dihydro-4-oxo-3-quinoléincarboxylique de point de fusion 182-186 °C.

Données spectroscopiques :

$^{1}$H RMN, δ ,J = Hz,[DMSO-TFA]; 1,35 (d,J = 6Hz,3H); 3,55 (m, 1H); 3,95 (m,1H); 4,95 (m,2H) ; 7,3 (m,3H); 7,8 (d, J = 13Hz,1H); 8,15 (é,2H); 8,6 (s,1H).

IR(KBr).- 2930, 1622, 1509, 1445 $cm^{-1}$.

Exemple 31. - Préparation de l'acide 8-chloro-1-(2,4-difluorophényl)-6-fluoro-7-(3-méthyl-3-amino-1-azétidinyl)-1,4-dihydro-4-oxo-3-quinoléincarboxylique.

Par une procédure tout à fait semblable à celle décrite dans l'exemple B, on obtient l'acide 8-chloro-1-(2,4-difluorophényl)-6-fluoro-7-(3-méthyl-3-amino-1-azétidinyl)-1,4-dihydro-4-oxo-3-quinoléincarboxylique de point de fusion 254-258 °C.

Données spectroscopiques :

$^{1}$H RMN, δ ,J = Hz,[DMSO-TFA]; 1,53 (s,3H); 4,47 (m,4H); 7,56 (m,3H); 7,89 (d,J = 13Hz,1H); 8,42 (é,2H); 8,57 (s,1H).

IR(KBr).- 2932, 1623, 1509, 1448 $cm^{-1}$.

Exemple 32. - Préparation de l'acide (±)-8-chloro-1-(2-fluoroéthyl)-6-fluoro-7-(trans-2-méthyl-3-amino-1-azétidinyl)-1,4-dihydro-4-oxo-3-quinoléincarboxylique.

Par une procédure tout à fait semblable à celle décrite dans l'exemple B, on obtient l'acide (±)-8-chloro -1-(2-fluoroéthyl)-6-fluoro-7-(trans-2-méthyl-3-amino-1-azétidinyl)-1,4-dihydro-4-oxo-3-quinoléincarboxylique de point de fusion 232-236 °C.

Données spectroscopiques :

$^{1}$H RMN, δ ,J = Hz,[DMSO-TFA]; 1,5 (d,J = 6Hz,3H); 3,7 (m, 1H); 4 (m,1H); 4,5 (m,1H); 5,0 (m,5H); 7,9 (d,J = 13Hz, 1H); 8,4 (é,2H); 8,45 (s,1H).

IR(KBr).- 2940, 1631, 1439, 1302 $cm^{-1}$.

Exemple 33. - Préparation de l'acide 8-chloro-1-(2-fluoroéthyl)-6-fluoro-7-(3-méthyl-3-amino-1-azétidinyl)-1,4-dihydro-4-oxo-3-quinoléincarboxylique.

Par une procédure tout à fait semblable à celle décrite dans l'exemple B, on obtient l'acide 8-chloro-1-(2-fluoroéthyl)-6-fluoro-7-(3-méthyl-3-amino-1-azétidinyl)-1,4-dihydro-4-oxo-3-quinoléincarboxyique de point de fusion 275-277 °C.

Données spectroscopiques :

$^{1}$H RMN, δ ,J = Hz,[DMSO-TFA]; 1,56 (s,3H); 4,52 (m,5H); 5,0 (m,2H); 5,3 (m,1H); 7,8 (d,J = 13Hz,1H); 8,5 (é,2H); 8,8 (s,1H).

IR(KBr).- 2930, 1634, 1611, 1445, 1333 $cm^{-1}$.

Exemple 34. - Préparation de l'acide (±)-8-chloro-6-fluoro-1-(4-fluorophényl)-7-(trans-2-méthyl-3-amino-1-azétidinyl)-1,4-dihydro-4-oxo-3-quinoléincarboxylique.

Par une procédure tout à fait semblable à celle décrite dans l'exemple B, on obtient l'acide (±)-8-chloro-6-fluoro-1-(4-fluorophényl)-7-(trans-2-méthyl-3-amino-1-azétidinyl)-1,4-dihydro-4-oxo-3-quinoléincarboxylique. de point de fusion 245-247 °C.

Données spectroscopiques :

$^{1}$H RMN, δ , J = Hz,[DMSO-TFA]; 1,38 (d,J = 6Hz,3H); 3,60 (m, 1H); 4,0 (m,1H) ; 4,85 (m,2H) ; 7,35 (m, 4H); 7,9 (d,J = 13Hz,1H) ; 8,30 (é,2H); 8,48 (s,1H).

IR(KBr).- 1727, 1620, 1505, 1432 $cm^{-1}$.

Exemple 35. - Préparation de l'acide 8-chloro-6-fluoro-1-(4-fluorophényl)-7-(3-méthyl-3-amino-1-azétidinyl)-1,4-dihydro-4-oxo-3-quinoléincarboxylique.

Par une procédure tout à fait semblable à celle décrite dans l'exemple B, on obtient l'acide 8-chloro-6-fluoro-1-(4-fluorophényl)-7-(3-méthyl-3-amino-1-azétidinyl)-1,4-dihydro-4-oxo-3-quinoléincarboxylique de point de fusion 256-259 °C.

Données spectroscopiques :

$^{1}$H RMN, δ ,J = Hz,[DMSO-TFA]; 1,51 (s,3H); 4,43 (m,4H); 7,41 (m,4H); 7,88 (d,J = 13Hz,1H); 8,36 (é,2H); 8,46 (s,1H).

IR(KBr).- 2940, 1620, 1441 $cm^{-1}$.

Exemple 36 . - Préparation de l'acide (±)-6-fluoro-1-(2-fluoroéthyl)-7-(trans-2-méthyl-3-amino-1-azétidinyl)-1,4-dihydro-4-oxo-1,8-naphtyridine-3-carboxylique.

Par une procédure tout à fait semblable à celle décrite dans l'exemple B, on obtient l'acide (±)-6-fluoro -1-(4-fluoroéthyl)-7-(trans-2-méthyl-3-amino-1-azétidinyl)-1,4-dihydro-4-oxo-1,8-naphtyridine-3-carboxylique de point de fusion 268-271 °C.

Données spectroscopiques :

$^{1}$H RMN, δ ,J = Hz,[DMSO-TFA]; 1,3 (d,J = 6Hz,3H); 3,6 (m,1H); 4 (m,1H); 4,6 (m,1H); 5,1 (m,5H); 7,81 (d,J = 11,5Hz,1H); 8,25 (é,2H); 8,79 (s,1H).

IR(KBr).- 1631, 1445, 1336 $cm^{-1}$.

Exemple 37 - Préparation de l'acide 6-fluoro-1-(2-fluoroéthyl)-7-(3-méthyl-3-amino-1-azétidinyl)-1,4-dihydro-4-oxo-1,8-naphtyridine-3-carboxylique.

Par une procédure tout à fait semblable à celle décrite dans l'exemple B, on obtient l'acide 6-fluoro-1-(2-fluoroéthyl)-7-(3-méthyl-3-amino-1-azétidinyl)-1,4-dihydro-4-oxo-1,8-naphtyridine-3-carboxylique de point de fusion 279-286 °C.

Données spectroscopiques :

$^{1}$H RMN, δ ,J = Hz,[DMSO-TFA]; 1,53 (s,3H); 4,4 (m,6H); 5,2 (m,2H); 8,09 (d,J = 11,5Hz,1H); 8,23 (é,2H); 8,8 (s,1H).

IR(KBr).- 1633, 1445, 1315 $cm^{-1}$.

Exemple 38. - Préparation de l'acide (±)-6-fluoro-1-(4-fluorophényl)-7-(trans-2-méthyl-3-amino-1-azétidinyl)-1,4-dihydro-4-oxo-1,8-naphtyridine-3-carboxylique.

Par une procédure tout à fait semblable à celle décrite dans l'exemple B, on obtient l'acide (±)-6-fluoro-1-(4-fluorophényl)-7-(trans-2-méthyl-3-amino-1-azétidinyl)-1,4-dihydro-4-oxo-1,8-naphtyridine-3-carboxylique de point de fusion 239-244 ° C.

Données spectroscopiques :

$^1$H RMN, $\delta$ ,J = Hz,[DMSO-TFA]; 1,17 (d,J = 6Hz,3H); 3,7 (m, 1H); 4,2 (m,2H); 4,4 (m,1H); 7,45 (m,4H); 8,12 (d, J = 11,5Hz,1H); 8,2 (é,2H); 8,67 (s,1H).

IR(KBr).- 1726, 1630, 1504, 1448 $cm^{-1}$.

Exemple 39. - Préparation de l'acide 6-fluoro-1-(4-fluorophényl)-7-(3-méthyl-3-amino-1-azétidinyl)-1,4-dihydro-4-oxo-1,8-naphtyridine-3-carboxylique.

Par une procédure tout à fait semblable à celle décrite dans l'exemple B, on obtient l'acide 6-fluoro-1-(4-fluorophényl)-7-(3-méthyl-3-amino-1-azétidinyl)-1,4-dihydro-4-oxo-1,8-naphtyridine-3-carboxylique de point de fusion 258-260 ° C.

Données spectroscopiques :

$^1$H RMN, $\delta$ ,J = Hz,[DMSO-TFA]; 1,52 (s,3H); 4,12 (m,4H); 7,4 (m,4H); 8,1 (d,J = 11,5Hz,1H); 8,31 (é,2H); 8,64 (s,1H).

IR(KBr).- 2935, 1631, 1460 $cm^{-1}$.

Exemple 40. - Préparation de l'acide (-)-1-2,4-difluorophényl)-6,8-difluoro-7-(3-(R)-amino-2-(S-méthyl-1-azétidinyl)-1,4-dihydro-4-oxo-3-quinoléincarboxylique.

Par une procédure tout à fait semblable à celle décrite dans l'exemple B, on obtient l'acide (-)-1-2,4-difluorophényl)-6,8-difluoro-7-(3-(R)-amino-2-(S-méthyl-1-azétidinyl)-1,4-dihydro-4-oxo-3-quinoléincarboxylique de point de fusion 200-204 ° C.

$[\alpha]_D^{20}$ = -14,0 (c = 0,30, NaOH 0,5N)

Données spectroscopiques :

$^1$H RMN, $\delta$ ,J = Hz,(DMSO-TFA]; 1,4 (d,J = 6Hz,3H); 3,65 (m,1H); 4,1 (m,1H); 4,6 (m,2H); 7,81 (m,4H); 8,34 (é,2H); 8,61 (s,1H).

IR(KBr).- 1619, 1509, 1474 $cm^{-1}$.

ACTIVITE BIOLOGIQUE

L'activité pharmacologique antimicrobienne de ces composés a été étudié selon les références exposées ci-dessous.

Activité pharmacologique antimicrobienne (G. L. Daquet et Y. A. Chabbect, Techniques en bactériologie, Vol. 3, Flammarion Médecine-Sciences, Paris, 1972, et W.B. Hugo et A. D. Russell, Pharmaceutical Microbiology, Blackwell Scientific Publications, London, 1977.

Milieu de culture et solvant

- Agar d'antibiotiques n° 1 (Oxoid CM 327)
- Bouillon de triptone-soja (Oxoid CM 129)
- Solution Physiologique Ringer 1/4 (Oxoid BR 52)
- Agar Dextrose (BBL 11165)

Microorganismes

"Bacillus subtilis" ATCC 6633
"Citrobacter Freundii" ATCC 112606
"Enterobacter aerogenes" ATCC 15038
"Enterobacter cloacae" ATCC 23355
"Bacilus cereus" ATCC 1178
"Escherichia coli" ATCC 10799
"Escherichia coli" ATCC 23559

"Klebsiella pneumonia" ATCC 10031
"Proteus vulgaris" ATCC 8427
"Morg. morganii" ATCC 8019
"Pseudomonas aeruginosa" ATCC 9721
"Pseudomonas aeruginosa" ATCC 10145
"Salmonella tiphymurium" ATCC 14028
"Salmonella tiphymurium" ATCC 6539
"Serratia marcescens" ATCC 13880
"Shigella flexnerii" ATCC 12022
"Staphylococcus epidermis" ATCC 155-1
"Staphylococcus aureus" ATCC 25178
"Streptococcus faecalis" ATCC 10541

Préparation des inoculations.

Chacun des microorganismes est ensemencé par strie dans des tubes d'Agar d'antibiotiques n° 1, et mis en incubation pendant 20 heures à 37 °C. Ensuite, on prend une anse de culture, on ensemence dans un bouillon de Triptone-soja et on incube pendant 20 heures à 37 °C. On dilue à 1/4 la culture obtenue avec une solution physiologique Ringer, de façon à obtenir une suspension normalisée de $10^7$-$10^9$ µfc/ml pour chaque organisme.

Préparation du milieu contenant des dérivés de formule générale I

A partir d'une solution de 100 µg/ml dans de la soude 0,1 N, chaque produit est dilué dans l'Agar Dextrose (préalablement fondu et maintenu à 50 °C) par dilutions successives de façon à obtenir les concentrations suivantes : 64-32-16-8-4-2-1-0,5-0,125 µg de dérivé/ml du milieu.

Postérieurement, chaque concentration de chaque produit est répartie dans des boîtes de Petri de 10 cm de diamètre, à raison de 10 ml de milieu par boîte et autant de boîtes que de microorganismes à tester.

Une fois le milieu refroidi, les boîtes sont ensemencées avec les inoculations à raison de 0,4 ml d'inoculation par boîte. On les étend avec une anse de Driglasky et on recueille le surnageant. Les boîtes ensemencées sont incubées à 37 °C pendant 20 heures.

Les résultats obtenus sont décrites dans les tableaux suivants. L'activité des composés "in vitro" y est comparée à celle de la norfloxacine. Les concentrations sont données en µg/ml.

| MICROORGANISMES | Norfloxacin | EXEMPLES | |
|---|---|---|---|
| | | A | B |
| Bacillus subtilis ATCC 6633 | 0.25 | 0.12 | 0.06 |
| Bacillus cereus ATCC 11778 | 1.0 | 0.50 | 0.25 |
| Strep. faecalis ATCC 10541 | 1.0 | 1.00 | 2.00 |
| Staph. aureus ATCC 25178 | 2.0 | 0.50 | 0.25 |
| Staph. epidermidis ATCC 155-1 | 1.0 | 0.50 | 0.25 |
| Ps. aeruginosa ATCC 9721 | 0.5 | 1.00 | 4.00 |
| Ps. aeruginosa ATCC 10145 | 1.0 | 2.00 | 4.00 |
| Citr. freundii ATCC 11606 | 0.25 | 0.12 | 0.25 |
| Morg. morganii ATCC 8019 | 0.12 | 0.12 | 0.25 |
| Proteus vulgaris ATCC 8427 | 0.06 | 1.00 | 0.50 |
| Kleb. pneumoniae ATCC 10031 | 0.03 | 0.12 | 0.25 |
| Sal. typhimurium ATCC 14028 | 0.25 | 0.25 | 0.50 |
| Sal. typhi ATCC 6539 | 0.06 | 0.12 | 0.50 |
| Escherichia coli ATCC 10799 | 0.25 | 0.25 | 0.50 |
| Escherichia coli ATCC 23559 | 0.06 | 0.12 | 0.25 |
| Ent. aerogenes ATCC 15038 | 0.25 | 0.25 | 0.25 |
| Ent. cloacae ATCC 23355 | 0.06 | 0.12 | 0.25 |
| Serr. marcescens ATCC 13880 | 0.50 | 0.25 | 1.00 |
| Shigella flexnerii ATCC 12022 | 0.12 | 0.06 | 0.25 |

| MICROORGANISMES | EXEMPLES | |
|---|---|---|
| | 1 | 2 |
| Bacillus subtilis ATCC 6633 | ≤0.03 | ≤0.03 |
| Bacillus cereus ATCC 11778 | 0.12 | 0.06 |
| Strep. faecalis ATCC 10541 | 0.25 | 0.25 |
| Staph. aureus ATCC 25178 | 0.25 | 0.12 |
| Staph. epidermidis ATCC 155-1 | 0.12 | 0.12 |
| Ps. aeruginosa ATCC 9721 | 0.50 | 0.25 |
| Ps. aeruginosa ATCC 10145 | 0.50 | 0.50 |
| Citr. freundii ATCC 11606 | 0.06 | ≤0.03 |
| Morg. morganii ATCC 8019 | 0.06 | ≤0.03 |
| Proteus vulgaris ATCC 8427 | 0.12 | 0.06 |
| Kleb. pneumoniae ATCC 10031 | ≤0.03 | ≤0.03 |
| Sal. typhimurium ATCC 14028 | 0.06 | ≤0.03 |
| Sal. typhi ATCC 6539 | ≤0.03 | ≤0.03 |
| Escherichia coli ATCC 10799 | 0.06 | ≤0.03 |
| Escherichia coli ATCC 23559 | ≤0.03 | ≤0.03 |
| Ent. aerogenes ATCC 15038 | ≤0.03 | ≤0.03 |
| Ent. cloacae ATCC 23355 | ≤0.03 | ≤0.03 |
| Serr. marcescens ATCC 13880 | 0.06 | 0.06 |
| Shigella flexnerii ATCC 12022 | ≤0.03 | ≤0.03 |

| MICROORGANISMES | EXEMPLES |
|---|---|
| | 3 |
| Bacillus subtilis ATCC 6633 | ≤0.03 |
| Bacillus cereus ATCC 11778 | 1.0 |
| Strep. faecalis ATCC 10541 | 1.0 |
| Staph. aureus ATCC 25178 | 0.12 |
| Staph. epidermidis ATCC 155-1 | 0.06 |
| Ps. aeruginosa ATCC 9721 | 4.0 |
| Ps. aeruginosa ATCC 10145 | 2.0 |
| Citr. freundii ATCC 11606 | 0.12 |
| Morg. morganii ATCC 8019 | 0.25 |
| Proteus vulgaris ATCC 8427 | 0.25 |
| Kleb. pneumoniae ATCC 10031 | 0.06 |
| Sal. typhimurium ATCC 14028 | 0.25 |
| Sal. typhi ATCC 6539 | 0.06 |
| Escherichia coli ATCC 10799 | 0.25 |
| Escherichia coli ATCC 23559 | 0.06 |
| Ent. aerogenes ATCC 15038 | 0.50 |
| Ent. cloacae ATCC 23355 | 0.12 |
| Serr. marcescens ATCC 13880 | 1.0 |
| Shigella flexnerii ATCC 12022 | 0.06 |

| MICROORGANISMES | EXEMPLES 4 |
|---|---|
| Bacillus subtilis ATCC 6633 | ≤0.03 |
| Bacillus cereus ATCC 11778 | 0.06 |
| Strep. faecalis ATCC 10541 | 0.50 |
| Staph. aureus ATCC 25178 | 0.06 |
| Staph. epidermidis ATCC 155-1 | ≤0.03 |
| Ps. aeruginosa ATCC 9721 | 0.50 |
| Ps. aeruginosa ATCC 10145 | 0.50 |
| Citr. freundii ATCC 11606 | ≤0.03 |
| Morg. morganii ATCC 8019 | 0.06 |
| Proteus vulgaris ATCC 8427 | 0.12 |
| Kleb. pneumoniae ATCC 10031 | ≤0.03 |
| Sal. typhimurium ATCC 14028 | ≤0.03 |
| Sal. typhi ATCC 6539 | ≤0.03 |
| Escherichia coli ATCC 10799 | ≤0.03 |
| Escherichia coli ATCC 23559 | ≤0.03 |
| Ent. aerogenes ATCC 15038 | ≤0.03 |
| Ent. cloacae ATCC 23355 | ≤0.03 |
| Serr. marcescens ATCC 13880 | 0.12 |
| Shigella flexnerii ATCC 12022 | ≤0.03 |

| MICROORGANISMES | EXEMPLES | |
|---|---|---|
| | 5 | 6 |
| Bacillus subtilis ATCC 6633 | 0.06 | 0.06 |
| Bacillus cereus ATCC 11778 | 0.12 | 0.25 |
| Strep. faecalis ATCC 10541 | 1.00 | 1.00 |
| Staph. aureus ATCC 25178 | 0.12 | 0.25 |
| Staph. epidermidis ATCC 155-1 | 0.12 | 0.12 |
| Ps. aeruginosa ATCC 9721 | 1.00 | 1.00 |
| Ps. aeruginosa ATCC 10145 | 1.00 | 2.00 |
| Citr. freundii ATCC 11606 | 0.12 | 0.12 |
| Morg. morganii ATCC 8019 | 0.12 | 0.12 |
| Proteus vulgaris ATCC 8427 | 0.25 | 0.50 |
| Kleb. pneumoniae ATCC 10031 | 0.12 | ≤0.03 |
| Sal. typhimurium ATCC 14028 | 0.12 | 0.12 |
| Sal. typhi ATCC 6539 | 0.06 | 0.06 |
| Escherichia coli ATCC 10799 | 0.12 | 0.12 |
| Escherichia coli ATCC 23559 | 0.06 | 0.06 |
| Ent. aerogenes ATCC 15038 | 0.25 | 0.12 |
| Ent. cloacae ATCC 23355 | 0.06 | 0.06 |
| Serr. marcescens ATCC 13880 | 0.50 | 0.50 |
| Shigella flexnerii ATCC 12022 | 0.06 | 0.06 |

| MICROORGANISMES | EXEMPLES 7 |
|---|---|
| Bacillus subtilis ATCC 6633 | ≤0.03 |
| Bacillus cereus ATCC 11778 | ≤0.03 |
| Strep. faecalis ATCC 10541 | 0.06 |
| Staph. aureus ATCC 25178 | ≤0.03 |
| Staph. epidermidis ATCC 155-1 | ≤0.03 |
| Ps. aeruginosa ATCC 9721 | 0.12 |
| Ps. aeruginosa ATCC 10145 | 0.25 |
| Citr. freundii ATCC 11606 | ≤0.03 |
| Morg. morganii ATCC 8019 | ≤0.03 |
| Proteus vulgaris ATCC 8427 | 0.06 |
| Kleb. pneumoniae ATCC 10031 | ≤0.03 |
| Sal. typhimurium ATCC 14028 | ≤0.03 |
| Sal. typhi ATCC 6539 | ≤0.03 |
| Escherichia coli ATCC 10799 | ≤0.03 |
| Escherichia coli ATCC 23559 | ≤0.03 |
| Ent. aerogenes ATCC 15038 | ≤0.03 |
| Ent. cloacae ATCC 23355 | ≤0.03 |
| Serr. marcescens ATCC 13880 | 0.06 |
| Shigella flexnerii ATCC 12022 | ≤0.03 |

| MICROORGANISMES | EXEMPLES | | |
|---|---|---|---|
| | 8 | 9 | 10 |
| Bacillus subtilis ATCC 6633 | ≤0.03 | 0.06 | 0.03 |
| Bacillus cereus ATCC 11778 | 0.12 | 0.12 | 0.25 |
| Strep. faecalis ATCC 10541 | 2.0 | 1.0 | 1.0 |
| Staph. aureus ATCC 25178 | 0.12 | 0.12 | 0.12 |
| Staph. epidermidis ATCC 155-1 | 0.12 | 0.12 | 0.12 |
| Ps. aeruginosa ATCC 9721 | 2.0 | 1.0 | 1.0 |
| Ps. aeruginosa ATCC 10145 | 2.0 | 1.0 | 2.0 |
| Citr. freundii ATCC 11606 | 0.06 | 0.12 | 0.06 |
| Morg. morganii ATCC 8019 | 0.12 | 0.25 | 0.25 |
| Proteus vulgaris ATCC 8427 | 0.25 | 0.50 | 0.50 |
| Kleb. pneumoniae ATCC 10031 | ≤0.03 | 0.06 | ≤0.03 |
| Sal. typhimurium ATCC 14028 | 0.12 | 0.12 | 0.50 |
| Sal. typhi ATCC 6539 | ≤0.03 | 0.06 | ≤0.03 |
| Escherichia coli ATCC 10799 | 0.12 | 0.12 | ≤0.03 |
| Escherichia coli ATCC 23559 | 0.06 | 0.06 | 0.06 |
| Ent. aerogenes ATCC 15038 | 0.12 | 0.12 | 0.06 |
| Ent. cloacae ATCC 23355 | 0.12 | 0.06 | 0.06 |
| Serr. marcescens ATCC 13880 | 0.50 | 0.50 | 1.0 |
| Shigella flexnerii ATCC 12022 | 0.06 | 0.12 | ≤0.03 |

| MICROORGANISMES | EXEMPLES | | | | | |
|---|---|---|---|---|---|---|
| | 11 | 12 | 13 | 14 | 15 | 16 |
| Bacillus subtilis ATCC 6633 | 0.06 | 0.12 | 0.25 | 0.12 | 0.12 | 0.12 |
| Bacillus cereus ATCC 11778 | 0.25 | 0.50 | 1.0 | 0.25 | 0.50 | 0.25 |
| Strep. faecalis ATCC 10541 | 0.50 | 2.0 | 2.0. | 2.0 | 1.0 | 2.0 |
| Staph. aureus ATCC 25178 | 0.25 | 0.50 | 1.0 | 0.25 | 0.25 | 0.5 |
| Staph. epidermidis ATCC 155-1 | 0.25 | 0.50 | 1.0 | 0.12 | 0.25 | 0.25 |
| Ps. aeruginosa ATCC 9721 | 1.0 | 1.0 | 2.0 | 2.0 | 2.0 | 2.0 |
| Ps. aeruginosa ATCC 10145 | 1.0 | 1.0 | 2.0 | 2.0 | 4.0 | 4.0 |
| Citr. freundii ATCC 11606 | 0.12 | 0.06 | 0.25 | 0.12 | 0.25 | 0.12 |
| Morg. morganii ATCC 8019 | 0.06 | 0.06 | 0.12 | 0.25 | 0.12 | 0.12 |
| Proteus vulgaris ATCC 8427 | 0.25 | 0.50 | 2.0 | 0.50 | 0.50 | 0.50 |
| Kleb. pneumoniae ATCC 10031 | ≤0.03 | ≤0.03 | 0.12 | 0.12 | 0.06 | 0.03 |
| Sal. typhimurium ATCC 14028 | 0.06 | 0.12 | 0.50 | 0.25 | 0.50 | 0.50 |
| Sal. typhi ATCC 6539 | ≤0.03 | 0.06 | 0.25 | 0.12 | 0.25 | 0.50 |
| Escherichia coli ATCC 10799 | 0.12 | ≤0.03 | 0.25 | 0.12 | 0.25 | 0.50 |
| Escherichia coli ATCC 23559 | 0.06 | 0.06 | 0.12 | 0.12 | 0.25 | 0.25 |
| Ent. aerogenes ATCC 15038 | 0.06 | 0.12 | 0.25 | 0.12 | 0.50 | 0.25 |
| Ent. cloacae ATCC 23355 | 0.06 | 0.06 | 0.12 | 0.12 | 0.25 | 0.25 |
| Serr. marcescens ATCC 13880 | 0.50 | 0.50 | 0.50 | 0.50 | 0.50 | 0.50 |
| Shigella flexnerii ATCC 12022 | ≤0.03 | 0.06 | 0.12 | 0.12 | 0.12 | 0.12 |

| MICROORGANISMES | EXEMPLES | | | | | |
|---|---|---|---|---|---|---|
| | 17 | 18 | 19 | 20 | 21 | 22 |
| Bacillus subtilis ATCC 6633 | 0.12 | ≤0.03 | 0.06 | ≤0.03 | 0.25 | 0.06 |
| Bacillus cereus ATCC 11778 | 0.50 | 0.06 | 0.25 | 0.12 | 1.0 | 0.50 |
| Strep. faecalis ATCC 10541 | 2.0 | 0.25 | 2.0 | 1.0 | 2.0 | 1.00 |
| Staph. aureus ATCC 25178 | 0.25 | ≤0.03 | 0.50 | 0.12 | 1.0 | 0.50 |
| Staph. epidermidis ATCC 155-1 | 0.25 | ≤0.03 | 0.25 | 0.12 | 1.0 | 0.50 |
| Ps. aeruginosa ATCC 9721 | 2.0 | 0.25 | 2.0 | 0.50 | 4.0 | 2.00 |
| Ps. aeruginosa ATCC 10145 | 4.0 | 0.25 | 2.0 | 1.00 | 4.0 | 4.00 |
| Citr. freundii ATCC 11606 | 0.25 | ≤0.03 | 0.12 | 0.06 | 0.50 | 0.12 |
| Morg. morganii ATCC 8019 | 0.25 | ≤0.03 | 0.25 | 0.06 | 0.50 | 0.25 |
| Proteus vulgaris ATCC 8427 | 0.50 | 0.06 | 0.50 | 0.25 | 1.0 | 0.50 |
| Kleb. pneumoniae ATCC 10031 | 0.12 | ≤0.03 | 0.06 | ≤0.03 | 0.25 | 0.06 |
| Sal. typhimurium ATCC 14028 | 0.50 | ≤0.03 | 0.50 | 0.25 | 0.50 | 0.25 |
| Sal. typhi ATCC 6539 | 0.25 | ≤0.03 | 0.12 | 0.06 | 0.50 | 0.12 |
| Escherichia coli ATCC 10799 | 0.25 | ≤0.03 | 0.25 | 0.06 | 0.50 | 0.25 |
| Escherichia coli ATCC 23559 | 0.25 | ≤0.03 | 0.12 | 0.03 | 0.25 | 0.06 |
| Ent. aerogenes ATCC 15038 | 0.25 | ≤0.03 | 0.12 | 0.03 | 0.50 | 0.06 |
| Ent. cloacae ATCC 23355 | 0.25 | ≤0.03 | 0.12 | 0.03 | 0.25 | 0.06 |
| Serr. marcescens ATCC 13880 | 0.50 | 0.06 | 0.50 | 0.25 | 1.0 | 0.50 |
| Shigella flexnerii ATCC 12022 | 0.25 | ≤0.03 | 0.12 | ≤0.03 | 0.25 | 0.06 |

| MICROORGANISMES | EXEMPLES | | |
|---|---|---|---|
| | 23 | 24 | 25 |
| Bacillus subtilis ATCC 6633 | ≤0.03 | 0.12 | ≤0.03 |
| Bacillus cereus ATCC 11778 | 0.06 | 0.25 | 0.06 |
| Strep. faecalis ATCC 10541 | 0.12 | 1.00 | 0.25 |
| Staph. aureus ATCC 25178 | ≤0.03 | 0.12 | 0.12 |
| Staph. epidermidis ATCC 155-1 | ≤0.03 | 0.12 | 0.12 |
| Ps. aeruginosa ATCC 9721 | 0.25 | 1 | 0.25 |
| Ps. aeruginosa ATCC 10145 | 0.25 | 2 | 0.50 |
| Citr. freundii ATCC 11606 | ≤0.03 | 0.12 | ≤0.03 |
| Morg. morganii ATCC 8019 | ≤0.03 | 0.12 | ≤0.03 |
| Proteus vulgaris ATCC 8427 | 0.06 | 0.25 | 0.06 |
| Kleb. pneumoniae ATCC 10031 | ≤0.03 | 0.12 | ≤0.03 |
| Sal. typhimurium ATCC 14028 | ≤0.03 | 0.12 | ≤0.03 |
| Sal. typhi ATCC 6539 | ≤0.03 | 0.12 | ≤0.03 |
| Escherichia coli ATCC 10799 | ≤0.03 | 0.12 | ≤0.03 |
| Escherichia coli ATCC 23559 | ≤0.03 | 0.12 | ≤0.03 |
| Ent. aerogenes ATCC 15038 | ≤0.03 | 0.12 | ≤0.03 |
| Ent. cloacae ATCC 23355 | ≤0.03 | 0.12 | ≤0.03 |
| Serr. marcescens ATCC 13880 | 0.12 | 1.0 | 0.06 |
| Shigella flexnerii ATCC 12022 | ≤0.03 | 0.12 | ≤0.03 |

| MICROORGANISMES | EXEMPLES 26 |
|---|---|
| Bacillus subtilis ATCC 6633 | ≤0.03 |
| Bacillus cereus ATCC 11778 | 0.06 |
| Strep. faecalis ATCC 10541 | 0.25 |
| Staph. aureus ATCC 25178 | 0.12 |
| Staph. epidermidis ATCC 155-1 | 0.12 |
| Ps. aeruginosa ATCC 9721 | 0.25 |
| Ps. aeruginosa ATCC 10145 | 0.50 |
| Citr. freundii ATCC 11606 | ≤0.03 |
| Morg. morganii ATCC 8019 | ≤0.03 |
| Proteus vulgaris ATCC 8427 | 0.06 |
| Kleb. pneumoniae ATCC 10031 | ≤0.03 |
| Sal. typhimurium ATCC 14028 | ≤0.03 |
| Sal. typhi ATCC 6539 | ≤0.03 |
| Escherichia coli ATCC 10799 | ≤0.03 |
| Escherichia coli ATCC 23559 | ≤0.03 |
| Ent. aerogenes ATCC 15038 | ≤0.03 |
| Ent. cloacae ATCC 23355 | ≤0.03 |
| Serr. marcescens ATCC 13880 | 0.06 |
| Shigella flexnerii ATCC 12022 | ≤0.03 |

| MICROORGANISMES | EXEMPLES 27 |
|---|---|
| Bacillus subtilis ATCC 6633 | 0.06 |
| Bacillus cereus ATCC 11778 | 0.25 |
| Strep. faecalis ATCC 10541 | 1.00 |
| Staph. aureus ATCC 25178 | 0.12 |
| Staph. epidermidis ATCC 155-1 | 0.25 |
| Ps. aeruginosa ATCC 9721 | 1.00 |
| Ps. aeruginosa ATCC 10145 | 1.00 |
| Citr. freundii ATCC 11606 | 0.25 |
| Morg. morganii ATCC 8019 | 0.25 |
| Proteus vulgaris ATCC 8427 | 1.00 |
| Kleb. pneumoniae ATCC 10031 | 0.06 |
| Sal. typhimurium ATCC 14028 | 0.25 |
| Sal. typhi ATCC 6539 | 0.12 |
| Escherichia coli ATCC 10799 | 0.25 |
| Escherichia coli ATCC 23559 | 0.12 |
| Ent. aerogenes ATCC 15038 | 0.12 |
| Ent. cloacae ATCC 23355 | 0.12 |
| Serr. marcescens ATCC 13880 | 0.25 |
| Shigella flexnerii ATCC 12022 | 0.12 |

| MICROORGANISMES | EXEMPLES | | | | |
|---|---|---|---|---|---|
| | 28 | 29 | 30 | 31 | 32 |
| Bacillus subtilis ATCC 6633 | ≤0.03 | 0.06 | ≤0.03 | ≤0.03 | 0.06 |
| Bacillus cereus ATCC 11778 | ≤0.03 | 0.06 | ≤0.03 | 0.06 | 0.25 |
| Strep. faecalis ATCC 10541 | 0.06 | 0.25 | 0.50 | 0.25 | 0.50 |
| Staph. aureus ATCC 25178 | ≤0.03 | 0.06 | 0.12 | 0.12 | 0.12 |
| Staph. epidermidis ATCC 155-1 | ≤0.03 | 0.06 | 0.06 | 0.06 | 0.12 |
| Ps. aeruginosa ATCC 9721 | 0.06 | 0.25 | 1,00 | 1,00 | 0.50 |
| Ps. aeruginosa ATCC 10145 | 0.12 | 0.25 | 0.50 | 0.50 | 1.00 |
| Citr. freundii ATCC 11606 | ≤0.03 | ≤0.03 | 0.06 | 0.12 | ≤0.03 |
| Morg. morganii ATCC 8019 | ≤0.03 | ≤0.03 | 0.25 | 0.25 | ≤0.03 |
| Proteus vulgaris ATCC 8427 | ≤0.03 | 0.25 | 0.25 | 0.12 | 0.25 |
| Kleb. pneumoniae ATCC 10031 | ≤0.03 | ≤0.03 | ≤0.03 | ≤0.03 | ≤0.03 |
| Sal. typhimurium ATCC 14028 | ≤0.03 | ≤0.03 | 0.12 | 0.25 | 0.06 |
| Sal. typhi ATCC 6539 | ≤0.03 | ≤0.03 | 0.06 | 0.12 | 0.06 |
| Escherichia coli ATCC 10799 | ≤0.03 | ≤0.03 | ≤0.03 | ≤0.03 | ≤0.03 |
| Escherichia coli ATCC 23559 | ≤0.03 | ≤0.03 | ≤0.03 | ≤0.03 | ≤0.03 |
| Ent. aerogenes ATCC 15038 | ≤0.03 | ≤0.03 | 0.06 | 0.12 | 0.06 |
| Ent. cloacae ATCC 23355 | ≤0.03 | ≤0.03 | 0.06 | 0.12 | ≤0.03 |
| Serr. marcescens ATCC 13880 | ≤0.03 | 0.25 | 1.00 | 1.00 | 0.25 |
| Shigella flexnerii ATCC 12022 | 0.06 | ≤0.03 | ≤0.03 | ≤0.03 | ≤0.03 |

| MICROORGANISMES | EXEMPLES | | | |
|---|---|---|---|---|
| | 33 | 34 | 35 | 36 |
| Bacillus subtilis ATCC 6633 | 0.06 | 0.12 | 0.25 | 0.06 |
| Bacillus cereus ATCC 11778 | 0.25 | 0.25 | 0.25 | 0.25 |
| Strep. faecalis ATCC 10541 | 1.00 | 2.00 | 2.00 | 0.50 |
| Staph. aureus ATCC 25178 | 0.12 | 0.25 | 0.25 | 0.12 |
| Staph. epidermidis ATCC 155-1 | 0.12 | 0.12 | 0.25 | 0.12 |
| Ps. aeruginosa ATCC 9721 | 1.00 | 2.00 | 2.00 | 0.50 |
| Ps. aeruginosa ATCC 10145 | 1.00 | 2.00 | 2.00 | 1.00 |
| Citr. freundii ATCC 11606 | 0.06 | 0.12 | 0.12 | ≤0.03 |
| Morg. morganii ATCC 8019 | 0.06 | 0.25 | 0.25 | 0.12 |
| Proteus vulgaris ATCC 8427 | 0.25 | 0.50 | 0.50 | 0.25 |
| Kleb. pneumoniae ATCC 10031 | 0.06 | 0.12 | 0.12 | ≤0.03 |
| Sal. typhimurium ATCC 14028 | 0.06 | 0.25 | 0.25 | 0.06 |
| Sal. typhi ATCC 6539 | 0.06 | 0.12 | 0.12 | 0.06 |
| Escherichia coli ATCC 10799 | 0.06 | 0.12 | 0.12 | ≤0.03 |
| Escherichia coli ATCC 23559 | 0.06 | 0.12 | 0.12 | ≤0.03 |
| Ent. aerogenes ATCC 15038 | 0.06 | 0.12 | 0.12 | 0.06 |
| Ent. cloacae ATCC 23355 | 0.06 | 0.12 | 0.12 | ≤0.03 |
| Serr. marcescens ATCC 13880 | 0.50 | 0.50 | 0.50 | 0.25 |
| Shigella flexnerii ATCC 12022 | 0.06 | 0.12 | 0.12 | ≤0.03 |

| MICROORGANISMES | EXEMPLES | | |
|---|---|---|---|
| | 37 | 38 | 39 |
| Bacillus subtilis ATCC 6633 | 0.06 | 0.25 | 0.25 |
| Bacillus cereus ATCC 11778 | 0.25 | 0.25 | 0.25 |
| Strep. faecalis ATCC 10541 | 1.00 | 2.00 | 2.00 |
| Staph. aureus ATCC 25178 | 0.12 | 0.25 | 0.25 |
| Staph. epidermidis ATCC 155-1 | 0.12 | 0.25 | 0.25 |
| Ps. aeruginosa ATCC 9721 | 1.00 | 2.00 | 2.00 |
| Ps. aeruginosa ATCC 10145 | 1.00 | 2.00 | 2.00 |
| Citr. freundii ATCC 11606 | 0.03 | 0.12 | 0.12 |
| Morg. morganii ATCC 8019 | 0.03 | 0.25 | 0.25 |
| Proteus vulgaris ATCC 8427 | 0.25 | 0.50 | 0.50 |
| Kleb. pneumoniae ATCC 10031 | 0.03 | 0.12 | 0.12 |
| Sal. typhimurium ATCC 14028 | 0.06 | 0.25 | 0.25 |
| Sal. typhi ATCC 6539 | 0.06 | 0.25 | 0.25 |
| Escherichia coli ATCC 10799 | ≤0.03 | 0.12 | 0.12 |
| Escherichia coli ATCC 23559 | ≤0.03 | 0.12 | 0.12 |
| Ent. aerogenes ATCC 15038 | 0.06 | 0.12 | 0.12 |
| Ent. cloacae ATCC 23355 | ≤0.03 | 0.12 | 0.12 |
| Serr. marcescens ATCC 13880 | 0.50 | 0.50 | 0.50 |
| Shigella flexnerii ATCC 12022 | ≤0.03 | 0.12 | 0.12 |

| MICROORGANISMES | EXEMPLES | | |
|---|---|---|---|
| | 40 | | |
| Bacillus subtilis ATCC 6633 | 0.06 | | |
| Bacillus cereus ATCC 11778 | 0.12 | | |
| Strep. faecalis ATCC 10541 | 1.00 | | |
| Staph. aureus ATCC 25178 | 0.12 | | |
| Staph. epidermidis ATCC 155-1 | 0.12 | | |
| Ps. aeruginosa ATCC 9721 | 1.00 | | |
| Ps. aeruginosa ATCC 10145 | 0.50 | | |
| Citr. freundii ATCC 11606 | 0.06 | | |
| Morg. morganii ATCC 8019 | 0.25 | | |
| Proteus vulgaris ATCC 8427 | 0.50 | | |
| Kleb. pneumoniae ATCC 10031 | ≤0.03 | | |
| Sal. typhimurium ATCC 14028 | 0.12 | | |
| Sal. typhi ATCC 6539 | 0.12 | | |
| Escherichia coli ATCC 10799 | 0.06 | | |
| Escherichia coli ATCC 23559 | 0.06 | | |
| Ent. aerogenes ATCC 15038 | 0.06 | | |
| Ent. cloacae ATCC 23355 | 0.06 | | |
| Serr. marcescens ATCC 13880 | 0.50 | | |
| Shigella flexnerii ATCC 12022 | ≤0.03 | | |

En thérapeutique humaine, la dose d'administration est bien sûr fonction de la susceptibilité de la souche infective, de la nature de composé administré et de la voie d'administration. Elle sera généralement comprise entre environ 0,200 et environ 300 mg pour chaque kilogramme de poids et par jour. Les dérivés de l'invention seront, par exemple, administrés sous forme de comprimés, de solutions ou de suspensions, ou bien de gélules.

On indiquera ci-après, à titre d'exemples, deux formes galéniques particulières des dérivés objets de la présente invention.

| Exemple de formule par comprimé | |
|---|---|
| Composé de l'exemple 9 | 250 mg |
| Cellule microcristalline | 69 mg |
| Povidone | 15 mg |
| Amidon de blé | 36 mg |
| Dioxyde de silice colloïdale | 2 mg |
| Stéarate de magnésium | 3 mg |
| Poids comprimé | 375 mg |

| Exemple de formule par gélule | |
|---|---|
| Composé de l'exemple 9 | 250 mg |
| Glycéride polyoxyéthylénée | 85 mg |
| Behenate de glycérine | 15 mg |
| Excipient gélatine molle q.s. | 450 mg |

## Revendications

**1.** Les composés de la formule générale I :

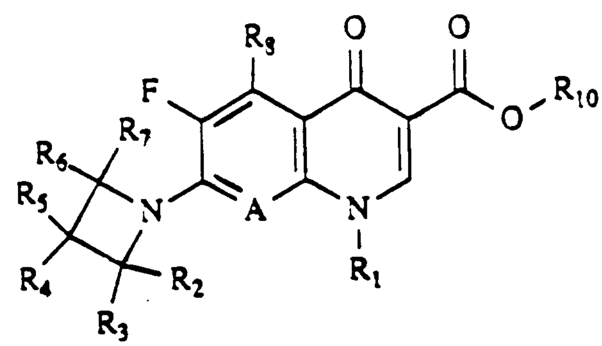

I

dans laquelle A représente un atome d'azote ou bien un atome de carbone avec un atome d'hydrogène attaché (C-H), ou bien un atome de carbone avec un halogène attaché (C-X), dans ce cas X représente un atome de chlore, de fluor ou de brome, ou bien un atome de carbone avec un radical hydroxy(C-OH),

$R_1$ représente un radical cycloalkyle inférieur, un radical halogénoalkyle inférieur, un radical aryle ou un radical aryle substitué, notamment par un ou plusieurs atome(s) de fluor,

$R_2$, $R_3$ et $R_7$ représentent un atome d'hydrogène,

$R_5$ et $R_6$, égaux ou différents, représentent un atome d'hydrogène ou un radical alkyle inférieur,

$R_4$ représente un radical amino, un radical alkylamino, un radical dialkylamino, ou un radical alkylcarboxamido, et dans ce dernier cas, le radical alkyle pouvant être substitué par un ou plusieurs halogènes,

$R_8$ représente un atome d'hydrogène, un radical nitro, un radical amino ou amine subsitué,

A et $R_1$ peuvent former ensemble une liaison représentée par un groupe $C-CH_2-CH_2-CHR_9-$ ou $C-O-CH_2-CHR_9-$ dans lesquels $R_9$ représente un atome d'hydrogène ou un radical alkyle inférieur, et dans ce dernier cas, on a un centre chiral avec une configuration "R" ou "S",

$R_{10}$ représente un atome d'hydrogène ou un radical alkyle inférieur de $C_1$ à $C_4$,

les substituants azétidiniques pouvant avoir, selon le nombre, la nature et la position relative des substituants, jusqu'à trois centre chiraux, chacun d'entre eux avec une configuration "R" ou "S", ainsi

que leurs sels d'acides minéraux tels les chlorhydrates, ou d'acides organiques tels les toluènesulfonates ou méthylsulfonates physiologiquement acceptables,

à l'exception des composés pour lesquels A représente un atome de carbone avec un atome d'hydrogène attaché (C-H) et $R_1$ représente un cycloalkyle inférieur,

à l'exception des composés pour lesquels $R_5$ et $R_6$ représentent un atome d'hydrogène et A représente un atome d'azote ou un atome de carbone avec un atome d'hydrogène attaché (C-H) ou un atome de carbone avec un halogène attaché (C-X), et X représente un atome de fluor, et

à la condition que lorsque $R_5$ représente un radical alkyle inférieur alors A représente un atome d'azote ou un atome de carbone avec un halogène attaché (C-X), et dans ce cas X représente un atome de chlore ou de brome, ou bien un atome de carbone avec un radical hydroxy (C-OH).

**2.** Les composés répondant à la formule générale I selon la revendication 1, sélectionnés parmi le groupe suivant :

- acide 1-cyclopropyl-6,8-difluoro-7-(trans-3-diméthylamino-2-méthyl-1-azétidinyl)-1,4-dihydro-4-oxo-3-quinoléincarboxylique.
- acide 1-cyclopropyl-6-fluoro-7-(trans-3-diméthylamino-2-méthyl-1-azétidinyl)-1,4-dihydro-4-oxo-3-quinoléincarboxylique.
- acide 1-cyclopropyl-6,8-difluoro-7-(trans-3-méthylamino-2-méthyl-1-azétidinyl)-1,4-dihydro-4-oxo-3-quinoléincarboxylique.
- acide 1-cyclopropyl-6,8-difluoro-7-(cis-3-amino-2-méthyl-1-azétidinyl)-1,4-dihydro-4-oxo-3-quinoléincarboxylique.
- acide 1-cyclopropyl-6,8-difluoro-7-(r-3-amino-3-trans-2-diméthyl-1-azetidinyl)-1,4-dihydro-4-oxo-3-quinoléincarboxylique.
- acide 5-amino-1-cyclopropyl-6,8-difluoro-7-(trans-3-amino-2-méthyl-1-azétidinyl)-1,4-dihydro-4-oxo-3-quinoléincarboxylique.
- acide 1-cyclopropyl-6,8-difluoro-7-(cis-3-amino-2-éthyl-1-azétidinyl)-1,4-dihydro-4-oxo-3-quinoléincarboxylique.
- acide 1-(2,4-difluorophényl)-6,8-difluoro-7-(trans-3-amino-2-méthyl-1-azétidinyl)-1,4-dihydro-4-oxo-3-quinoléincarboxylique.
- acide 1-(2,4-difluorophényl)-6-fluoro-7-(trans-3-amino-2-méthyl-1-azétidinyl)-1,4-dihydro-4-oxo-3-quinoléincarboxylique.
- acide 1-(4-fluorophényl)-6-fluoro-7-(trans-3-amino-2-méthyl-1-azetidinyl)-1,4-dihydro-4-oxo-3-quinoléincarboxylique.
- acide 1-(2-fluorophényl)-6,8-difluoro-7-(trans-3-amino-2-méthyl-1-azétidinyl)-1,4-dihydro-4-oxo-3-quinoléincarboxylique.
- acide 1-(2-fluoroéthyl)-6-fluoro-7-(trans-3-amino-2-méthyl-1-azétidinyl)-1,4-dihydro-4-oxo-3-quinoléincarboxylique.
- acide 1-(4-fluorophényl)-6,8-difluoro-7-(trans-3-amino-2-méthyl-1-azétidinyl)-1,4-dihydro-4-oxo-3-quinoléincarboxylique.
- acide (+)-1-cyclopropyl-6,8-difluoro-7-(3-(S)-amino-2-(R)-méthyl-1-azétidinyl)-1,4-dihydro-4-oxo-3-quinoléincarboxylique.
- acide (-)-1-cyclopropyl-6,8-difluoro-7-(3-(R)-amino-2-(S)-méthyl-1-azétidinyl)-1,4-dihydro-4-oxo-3-quinoléincarboxylique.
- acide (±)-8-chloro-1-cyclopropyl-6-fluoro-7-(trans-2-méthyl-3-amino-1-azétidinyl)-1,4-dihyro-4-oxo-3-quinoléincarboxylique.
- acide 8-chloro-1-cyclopropyl-6-fluoro-7-(3-méthyl-3-amino-1-azétidinyl)-1,4-dihydro-4-oxo-3-quinoléincarboxylique.
- acide (±)-8-chloro-1-(2,4-difluorophényl)-6-fluoro-7-(trans-2-méthyl-3-amino-1-azétidinyl)-1,4-dihydro-4-oxo-3-quinoléincarboxylique.
- acide 8-chloro-1-(2,4-difluorophényl)-6-fluoro-7-(3-methyl-3-amino-1-azétidinyl)-1,4-dihydro-4-oxo-3-quinoléincarboxylique.
- acide (±)-8-chloro-1-(2-fluorophényl)-6-fluoro-7-(trans-2-méthyl-3-amino-1-azétidinyl)-1,4-dihydro-4-oxo-3-quinoléincarboxylique.
- acide 8-chloro-1-(2-fluoroéthyl)-6-fluoro-7-(3-méthyl-3-amino-1-azétidinyl)-1,4-dihydro-4-oxo-3-quinoléincarboxylique
- acide (±)-8-chloro-6-fluoro-1-(4-fluorophényl)-7-(trans-2-méthyl-3-amino-1-azétidinyl)-1,4-dihydro-4-oxo-3-quinoléincarboxylique.

- acide 8-chloro-6-fluoro-1-(4-fluorophényl)-7-(3-méthyl-3-amino-1-azétidinyl)-1,4-dihydro-4-oxo-3-quinoléincarboxylique.
- acide (-)-1-(2,4-difluorophényl)-6,8-difluoro-7-(3-(R)-amino-2-(S)-méthyl-1-azétidinyl)-1,4-dihydro-4-oxo-3-quinoléincarboxylique.

**3.** Les composés répondant à la formule générale I selon la revendication 1, sélectionnés parmi le groupe suivant :
- acide 1-cyclopropyl-6-fluoro-7-(3-amino-3-méthyl-1-azétidinyl)-1,4-dihydro-4-oxo-1,8-naphtyridine-3-carboxylique.
- acide 1-cyclopropyl-6-fluoro-7-(trans-3-amino-2-méthyl-1-azétidinyl)-1,4-dihydro-4-oxo-1,8-naphtyridine-3-carboxylique.
- acide (-)-1-cyclopropyl-6-fluoro-7-(3-(S)-amino-2-(R)-méthyl-1-azétidinyl)-1,4-dihydro-4-oxo-1,8-naphtyridine-3-carboxylique.
- acide (+)-1-cyclopropyl-6-fluoro-7-(3-(R)-amino-2-(S)-méthyl-1-azétidinyl)-1,4-dihydro-4-oxo-1,8-naphtyridine-3-carboxylique.
- acide 1-cyclopropyl-6-fluoro-7-(3-méthyl-3-méthylamino-1-azétidinyl)-1,4-dihydro-4-oxo-1,8-naphtyridine-3-carboxylique.
- acide 6-fluoro-7-(trans-2-méthyl-3-amino-1-azétidinyl)-1-cyclopropyl-1,4-dihydro-4-oxo-1,8-naphtyridine-3-carboxylique.
- acide (±)-1-(2,4-difluorophényl)-6-fluoro-7-(trans-2-méthyl-3-amino-1-azétidinyl)-1,4-dihydro-4-oxo-1,8-naphtyridine-3-carboxylique.
- acide (±)-6-fluoro-1-(2-fluoroéthyl)-7-(trans-2-méthyl-3-amino-1-azétidinyl)-1,4-dihydro-4-oxo-1,8-naphtyridine-3-carboxylique.
- acide 6-fluoro-1-(2-fluoroéthyl)-7-(3-méthyl-3-amino-1-azétidinyl)-1,4-dihydro-4-oxo-1,8-naphtyridine-3-carboxylique.
- acide (±)-6-fluoro-1-(4-fluorophényl)-7-(trans-2-méthyl-3-amino-1-azétidinyl)-1,4-dihydro-4-oxo-1,8-naphtyridine-3-carboxylique.
- acide 6-fluoro-1-(4-fluorophényl)-7-(3-méthyl-3-amino-1-azétidinyl)-1,4-dihydro-4-oxo-1,8-naphtyridine-3-carboxylique.

**4.** Les composés répondant à la formule générale I selon la revendication 1, sélectionnés parmi le groupe suivant :
- acide (-)-(3S)-10-[3-(S)-amino-2-(R)-méthyl-1-azétidinyl]-9-fluoro-2,3-dihydro-3-méthyl-7-oxo-7H-pyrido[1,2,3-de][1,4]benzoxazine-6-carboxylique.
- acide (+)-(3R)-10-[3-(R)-amino-2-(S)-méthyl-1-azétidinyl]-9-fluoro-2,3-dihydro-3-méthyl-7-oxo-7H-pyrido [1,2,3-de][1,4]benzoxazine-6-carboxylique.
- acide (-)-(3S)-10-[3-(R)-amino-2-(S)-méthyl-1-azétidinyl]-9-fluoro-2,3-dihydro-3-méthyl-7-oxo-7H-pyrido[1,2,3-de][1,4]benzoxazine-6-carboxylique
- acide (+)-(3R)-10-[3-(S)-amino-2-(R)-méthyl-1-azétidinyl]-9-fluoro-2,3-dihydro-3-méthyl-7-oxo-7H-pyrido[1,2,3-de][1,4]benzoxozine-6-carboxylique.

**5.** Procédé de préparation de ces composés, selon l'une quelconque des revendications 1 à 4 et qui se caractérise par la réaction d'un composé de formule générale II

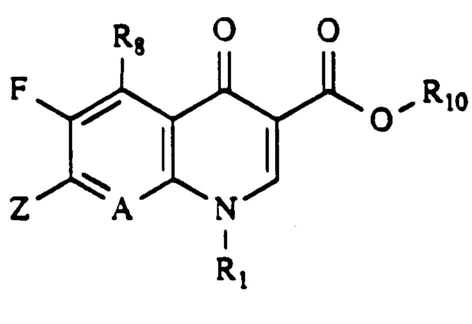

II

dans laquelle A, $R_1$, $R_8$ et $R_{10}$ ont les significations mentionnées précédemment et Z représente un atome d'halogène, de préférence un chlore ou un fluor, avec une azétidine, de formule générale III

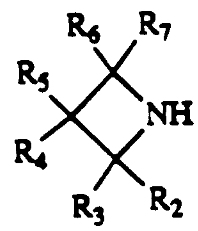

III

dans laquelle $R_2$, $R_3$, $R_4$, $R_5$, $R_6$ et $R_7$ ont les significations mentionnées précédemment.

**6.** A titre de médicaments, les dérivés de formule générale I et leurs sels thérapeutiquement acceptables, selon les revendications 1 à 4 , en particulier à titre de médicaments destinés au traitement de certain es maladies infectieuses.

**7.** Compositions pharmaceutiques, caractérisées par le fait qu'elles contiennent, outre un support pharmaceutiquement acceptable, au moins un dérivé de formule générale I ou l'un de ses sels physiologiquement acceptables, selon l'une des revendications 1 à 4.

**8.** Utilisation des dérivés de formule générale I et leurs sels physiologiquement acceptables, selon l'une des revendications 1 à 4, pour la fabrication de médicaments destinés au traitement des infections microbiennes.

## Claims

**1.** The compounds of the general formula I:

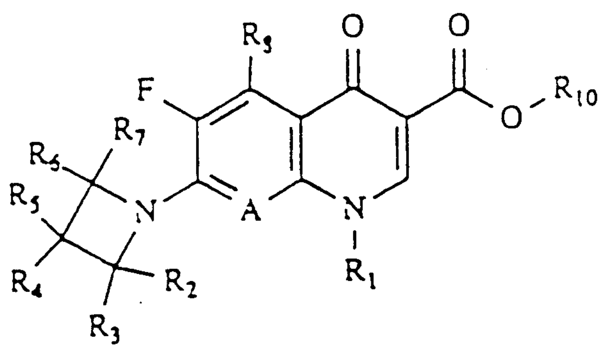

I

in which A represents a nitrogen atom, or alternatively a carbon atom with a hydrogen atom attached (C-H), or alternatively a carbon atom with a halogen attached (C-X) and in this case X represents a chlorine, fluorine or bromine atom, or alternatively a carbon atom with a hydroxyl radical (C-OH),

$R_1$ represents a lower cycloalkyl radical, a lower haloalkyl radical, an aryl radical or an aryl radical substituted, in particular, with one or more fluorine atom(s),

$R_2$, $R^3$ and $R_7$ represent a hydrogen atom,

$R_5$ and $R_6$, which may be the same or different, represent a hydrogen atom or a lower alkyl radical,

$R_4$ represents an amino radical, an alkylamino radical, a dialkylamino radical or an alkylcarboxamido radical and, in this latter case, it being possible for the alkyl radical to be substituted with one or more halogens,

$R_8$ represents a hydrogen atom, a nitro radical or an amino or substituted amino radical,

A and $R_1$ together can form a link represented by a group $C-CH_2-CH_2-CHR_9-$ or $C-O-CH_2-CHR_9-$ in which $R_9$ represents a hydrogen atom or a lower alkyl radical and, in this latter case, there is a chiral centre with an "R" or "S" configuration,

$R_{10}$ represents a hydrogen atom or a $C_1$ to $C_4$ lower alkyl radical,

it being possible for the azetidine substituents to have, depending on the number, nature and relative position of the substituents, up to three chiral centres, each of them with an "R" or "S"

configuration, as well as their physiologically acceptable salts with inorganic acids, such as the hydrochlorides, or with organic acids, such as the toluenesulphonates or methylsulphonates,

except for the compounds for which A represents a carbon atom with a hydrogen atom attached (C-H) and $R_1$ represents a lower cycloalkyl,

except for the compounds for which $R_5$ and $R_6$ represent a hydrogen atom and A represents a nitrogen atom or a carbon atom which a hydrogen atom attached (C-H) or a carbon atom with a halogen attached (C-X), and X represents a fluorine atom, and

on condition that, when $R_5$ represents a lower alkyl radical, then A represents a nitrogen atom or a carbon atom with a halogen attached (C-X), and in this case X represents a chlorine or bromine atom, or alternatively a carbon atom with a hydroxyl radical (C-OH).

**2.** The compounds corresponding to the general formula I according to Claim 1, selected from the following group:

- 1-cyclopropyl-6,8-difluoro-7-(trans-3-dimethylamino-2-methyl-1-azetidinyl)-1,4-dihydro-4-oxo-3-quinolinecarboxylic acid.
- 1-cyclopropyl-6-fluoro-7-(trans-3-dimethylamino-2-methyl-1-azetidinyl)-1,4-dihydro-4-oxo-3-quinolinecarboxylic acid.
- 1-cyclopropyl-6,8-difluoro-7-(trans-3-methylamino-2-methyl-1-azetidinyl)-1,4-dihydro-4-oxo-3-quinolinecarboxylic acid.
- 1-cyclopropyl-6,8-difluoro-7-(cis-3-amino-2-methyl-1-azetidinyl)-1,4-dihydro-4-oxo-3-quinolinecarboxylic acid.
- 1-cyclopropyl-6,8-difluoro-7-(r-3-amino-3-trans-2-dimethyl-1-azetidinyl)-1,4-dihydro-4-oxo-3-quinolinecarboxylic acid.
- 5-amino-1-cyclopropyl-6,8-difluoro-7-(trans-3-amino-2-methyl-1-azetidinyl)-1,4-dihydro-4-oxo-3-quinolinecarboxylic acid.
- 1-cyclopropyl-6,8-difluoro-7-(cis-3-amino-2-ethyl-1-azetidinyl)-1,4-dihydro-4-oxo-3-quinolinecarboxylic acid.
- 1-(2,4-difluorophenyl)-6,8-difluoro-7-(trans-3-amino-2-methyl-1-azetidinyl)-1,4-dihydro-4-oxo-3-quinolinecarboxylic acid.
- 1-(2,4-difluorophenyl)-6-fluoro-7-(trans-3-amino-2-methyl-1-azetidinyl)-1,4-dihydro-4-oxo-3-quinolinecarboxylic acid.
- 1-(4-fluorophenyl)-6-fluoro-7-(trans-3-amino-2-methyl-1-azetidinyl)-1,4-dihydro-4-oxo-3-quinolinecarboxylic acid.
- 1-(2-fluoroethyl)-6,8-difluoro-7-(trans-3-amino-2-methyl-1-azetidinyl)-1,4-dihydro-4-oxo-3-quinolinecarboxylic acid.
- 1-(2-fluoroethyl)-6-fluoro-7-(trans-3-amino-2-methyl-1-azetidinyl)-1,4-dihydro-4-oxo-3-quinolinecarboxylic acid.
- 1-(4-fluorophenyl)-6,8-difluoro-7-(trans-3-amino-2-methyl-1-azetidinyl)-1,4-dihydro-4-oxo-3-quinolinecarboxylic acid.
- (+)-1-cyclopropyl-6,8-difluoro-7-[(2R,3S)-3-amino-2-methyl-1-azetidinyl]-1,4-dihydro-4-oxo-3-quinolinecarboxylic acid.
- (-)-1-cyclopropyl-6,8-difluoro-7-[(2S,3R)-3-amino-2-methyl-1-azetidinyl]-1,4-dihydro-4-oxo-3-quinolinecarboxylic acid.
- (±)-8-chloro-1-cyclopropyl-6-fluoro-7-(trans-2-methyl-3-amino-1-azetidinyl)-1,4-dihydro-4-oxo-3-quinolinecarboxylic acid>
- 8-chloro-1-cyclopropyl-6-fluoro-7-(3-methyl-3-amino-1-azetidinyl)-1,4-dihydro-4-oxo-3-quinolinecarboxylic acid.
- (±)-8-chloro-1-(2,4-difluorophenyl)-6-fluoro-7-(trans-2-methyl-3-amino-1-azetidinyl)-1,4-dihydro-4-oxo-3-quinolinecarboxylic acid.
- 8-chloro-1-(2,4-difluorophenyl)-6-fluoro-7-(3-methyl-3-amino-1-azetidinyl)-1,4-dihydro-4-oxo-3-quinolinecarboxylic acid.
- (±)-8-chloro-1-(2-fluoroethyl)-6-fluoro-7-(trans-2-methyl-3-amino-1-azetidinyl)-1,4-dihydro-4-oxo-3-quinolinecarboxylic acid.
- 8-chloro-1-(2-fluoroethyl)-6-fluoro-7-(3-methyl-3-amino-1-azetidinyl)-1,4-dihydro-4-oxo-3-quinolinecarboxylic acid.
- (±)-8-chloro-6-fluoro-1-(4-fluorophenyl)-7-(trans-2-methyl-3-amino-1-azetidinyl)-1,4-dihydro-4-oxo-3-quinolinecarboxylic acid.

- 8-chloro-6-fluoro-1-(4-fluorophenyl)-7-(3-methyl-3-amino-1-azetidinyl)-1,4-dihydro-4-oxo-3-quinolinecarboxylic acid.
- (-)-1-(2,4-difluorophenyl)-6,8-difluoro-7-[(2S,3R)-3-amino-2-methyl-1-azetidinyl]-1,4-dihydro-4-oxo-3-quinolinecarboxylic acid.

**3.** The compounds corresponding to the general formula I according to Claim 1, selected from the following group:
- 1-cyclopropyl-6-fluoro-7-(3-amino-3-methyl-1-azetidinyl)-1,4-dihydro-4-oxo-1,8-napthyridine-3-carboxylic acid.
- 1-cyclopropyl-6-fluoro-7-(trans-3-amino-2-methyl-1-azetidinyl)-1,4-dihydro-4-oxo-1,8-naphthyridine-3-carboxylic acid.
- (-)-1-cyclopropyl-6-fluoro-7-[(2R,3S)-3-amino-2-methyl-1-azetidinyl]-1,4-dihydro-oxo-1,8-naphthyridine-3-carboxylic acid.
- (+)-1-cyclopropyl-6-fluoro-7-[(2S,3R)-3-amino-2-methyl-1-azetidinyl]-1,4-dihydro-4-oxo-1,8-naphthyridine-3-carboxylic acid.
- 1-cyclopropyl-6-fluoro-7-(3-methyl-3-methylamino-1-azetidinyl)-1,4-dihydro-4-oxo-1,8-naphthyridine-3-carboxylic acid.
- 6-fluoro-7-(trans-2-methyl-3-amino-1-azetidinyl)-1-cyclopropyl-1,4-dihydro-4-oxo-1,8-naphthyridine-3-carboxylic acid.
- (±)-1-(2,4-difluorophenyl)-6-fluoro-7-(trans-2-methyl-3-amino-1-azetidinyl)-1,4-dihydro-4-oxo-1,8-naphthyridine-3-carboxylic acid.
- (±)-6-fluoro-1-(2-fluoroethyl)-7-(trans-2-methyl-3-amino-1-azetidinyl)-1,4-dihydro-4-oxo-1,8-naphthyridine-3-carboxylic acid.
- 6-fluoro-1-(2-fluoroethyl)-7-(3-methyl-3-amino-1-azetidinyl)-1,4-dihydro-4-oxo-1,8-naphthyridine-3-carboxylic acid.
- (±)-6-fluoro-1-(4-fluorophenyl)-7-(trans-2-methyl-3-amino-1-azetidinyl)-1,4-dihydro-4-oxo-1,8-naphthyridine-3-carboxylic acid.
- 6-fluoro-1-(4-fluorophenyl)-7-(3-methyl-3-amino-1-azetidinyl)-1,4-dihydro-4-oxo-1,8-naphthyridine-3-carboxylic acid.

**4.** The compounds corresponding to the general formula I according to Claim 1, selected from the following group:
- (3S)-(-)-10-[(2R,3S)-3-amino-2-methyl-1-azetidinyl]-9-fluoro-2,3-dihydro-3-methyl-7-oxo-7H-pyrido-[1,2,3-de][1,4]benzoxazine-6-carboxylic acid.
- (3R)-(+)-10-[(2S,3R)-3-amino-2-methyl-1-azetidinyl]-9-fluoro-2,3-dihydro-3-methyl-7-oxo-7H-pyrido[1,2,3-de][1,4]benzoxazine-6-carboxylic acid.
- (3S)-(-)-10-[(2S,3R)-3-amino-2-methyl-1-azetidinyl]-9-fluoro-2,3-dihydro-3-methyl-7-oxo-7H-pyrido-[1,2,3-de][1,4]benzoxazine-6-carboxylic acid.
- (3R)-(+)-10-[(2R,3S)-3-amino-2-methyl-1-azetidinyl]-9-fluoro-2,3-dihydro-3-methyl-7-oxo-7H-pyrido[1,2,3-de][1,4]benzoxazine-6-carboxylic acid.

**5.** Process for preparing these compounds according to either of Claims 1 to 4, and which is characterized by the reaction of a compound of general formula II

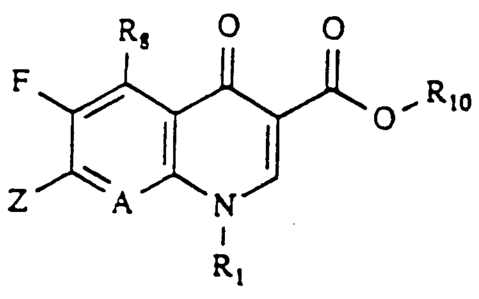

II

in which A, $R_1$, $R_8$ and $R_{10}$ have the meanings stated above and Z represents a halogen atom, preferably a chlorine or a fluorine, with an azetidine of general formula III

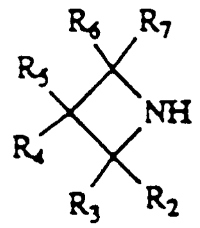 III

in which $R_2$, $R_3$, $R_4$, $R_5$, $R_6$ and $R_7$ have the meanings stated above.

**6.** By way of medicinal products, the derivatives of general formula I and their therapeutically acceptable salts, according to Claims 1 to 4, especially by way of medicinal products intended for the treatment of certain infectious diseases.

**7.** Pharmaceutical compositions, characterized in that they contain, in addition to a pharmaceutically acceptable vehicle, at least one derivative of general formula I or one of its physiologically acceptable salts, according to one of Claims 1 to 4.

**8.** Use of the derivatives of general formula I and their physiologically acceptable salts, according to one of Claims 1 to 4, for the manufacture of medicinal products intended for the treatment of microbial infections.

## Patentansprüche

**1.** Verbindungen der allgemeinen Formel (I):

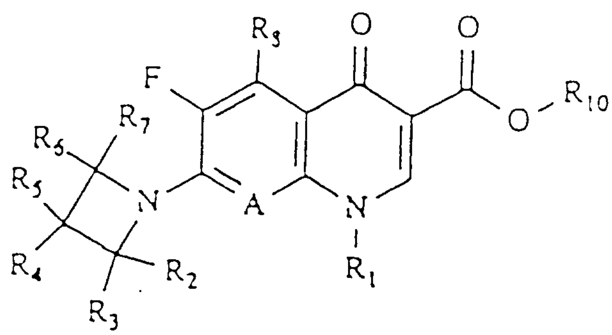 (I)

worin bedeuten:

| | |
|---|---|
| A | ein Stickstoffatom oder auch ein Kohlenstoffatom mit einem daran gebundenen Wasserstoffatom (C-H) oder auch ein Kohlenstoffatomen mit einem daran gebundenen Halogenatom (C-X), wobei in diesem Falle X für ein Chlor-, Fluor- oder Bromatom steht, oder auch ein Kohlenstoffatom mit einem Hydroxy-Rest (C-OH), |
| $R_1$ | einen niederen Cycloalkylrest, einen niederen Halogenalkylrest, einen Arylrest oder einen substituierten Arylrest, der insbesondere durch ein oder mehr Fluoratome substituiert ist, |
| $R_2$, $R_3$ und $R_7$ | ein Wasserstoffatom, |
| $R_5$ und $R_6$, | die gleich oder verschieden sind, ein Wasserstoffatom oder einen niederen Alkylrest, |
| $R_4$ | einen Aminorest, einen Alkylaminorest, einen Dialkylaminorest oder einen Alkylcarboxamidorest, wobei im zuletzt genannten Fall der Alkylrest durch ein oder mehr Halogenatome substituiert sein kann, |
| $R_8$ | ein Wasserstoffatom, einen Nitrorest, einen Aminorest oder einen substituierten Aminorest, wobei A und $R_1$ gemeinsam eine Bindung bilden können, dargestellt durch die Gruppe $C-CH_2-CH_2-CHR_9-$ oder $C-O-CH_2-CHR_9-$, worin $R_9$ für ein |

Wasserstoffatom oder einen niederen Alkylrest steht und wobei im letzteren Fall ein chirales Zentrum mit der Konfiguration "R" oder "S" vorliegt,

$R_{10}$ ein Wasserstoffatom oder einen niederen Alkylrest mit 1 bis 4 Kohlenstoffatomen wobei die Azetidin-Substituenten entsprechend der Anzahl, der Art und der relativen Position der Substituenten bis zu 3 chirale Zentren aufweisen können, von denen jedes eine "R" oder "S" Konfiguration hat,

sowie ihre Salze mit physiologisch akzeptablen Mineralsäuren wie die Hydrochloride oder mit physiologisch akzeptablen organischen Säuren wie die Toluolsulfonate oder Methylsulfonate,
ausgenommen die Verbindungen, in denen A ein Kohlenstoffatom mit einem daran gebundenen Wasserstoffatom (C-H) und $R_1$ einen niederen Cycloalkylrest darstellen, und ausgenommen die Verbindungen, in denen $R_5$ und $R_6$ ein Wasserstoffatom und A ein Stickstoffatom oder ein Kohlenstoffatom mit einem daran gebundenen Wasserstoffatom (C-H) oder ein Kohlenstoffatomen mit einem daran gebundenen Halogenatom (C-X) und X ein Fluoratom darstellen, und mit der Maßgabe, daß dann, wenn $R_5$ einen niederen Alkylrest darstellt, A dann für ein Stickstoffatom oder ein Kohlenstoffatom mit einem daran gebundenen Halogenatom (C-X) wobei in diesem Fall X ein Chlor- oder Bromatom ist, oder auch für ein Kohlenstoffatom mit einem Hydroxyrest (C-OH) steht.

**2.** Verbindungen der allgemeinen Formel (I) nach Anspruch 1, ausgewählt aus der folgenden Gruppe:
- 1-Cyclopropyl-6,8-difluoro-7-(trans-3-dimethylamino-2-methyl-1-azetidinyl)-1,4-dihydro-4-oxo-3-chinolincarbonsäure,
- 1-Cyclopropyl-6-fluoro-7-(trans-3-dimethylamino-2-methyl-1-azetidinyl)-1,4-dihydro-4-oxo-3-chinolincarbonsäure,
- 1-Cyclopropyl-6,8-difluoro-7-(trans-3-methylamino-2-methyl-1-azetidinyl)-1,4-dihydro-4-oxo-3-chinolincarbonsäure,
- 1-Cyclopropyl-6,8-difluoro-7-(cis-3-amino-2-methyl-1-azetidinyl)-1,4-dihydro-4-oxo-3-chinolincarbonsäure,
- 1-Cyclopropyl-6,8-difluoro-7-(r-3-amino-3-trans-2-dimethyl-1-azetidinyl)-1,4-dihydro-4-oxo-3-chinolincarbonsäure,
- 5-Amino-1-cyclopropyl-6,8-difluoro-7-(trans-3-amino-2-methyl-1-azetidinyl)-1,4-dihydro-4-oxo-3-chinolincarbonsäure,
- 1-Cyclopropyl-6,8-difluoro-7-(cis-3-amino-2-ethyl-1-azetidinyl)-1,4-dihydro-4-oxo-3-chinolincarbonsäure,
- 1-(2,4-Difluorophenyl)-6,8-difluoro-7-(trans-3-amino-2-methyl-1-azetidinyl)-1,4-dihydro-4-oxo-3-chinolincarbonsäure,
- 1-(2,4-Difluorophenyl)-6-fluoro-7-(trans-3-amino-2-methyl-1-azetidinyl)-1,4-dihydro-4-oxo-3-chinolincarbonsäure,
- 1-(4-Fluorophenyl)-6-fluoro-7-(trans-3-amino-2-methyl-1-azetidinyl)-1,4-dihydro-4-oxo-3-chinolincarbonsäure,
- 1-(2-Fluoroethyl)-6,8-difluoro-7-(trans-3-amino-2-methyl-1-azetidinyl)-1,4-dihydro-4-oxo-3-chinolincarbonsäure,
- 1-(2-Fluoroethyl)-6-fluoro-7-(trans-3-amino-2-methyl-1-azetidinyl)-1,4-dihydro-4-oxo-3-chinolincarbonsäure,
- 1-(4-Fluorophenyl)-6,8-difluoro-7-(trans-3-amino-2-methyl-1-azetidinyl)-1,4-dihydro-4-oxo-3-chinolincarbonsäure,
- (+)-1-Cyclopropyl-6,8-difluoro-7-(3-(S)-amino-2-(R)-methyl-1-azetidinyl)-1,4-dihydro-4-oxo-3-chinolincarbonsäure,
- (-)-1-Cyclopropyl-6,8-difluoro-7-(3-(R)-amino-2-(S)-methyl-1-azetidinyl)-1,4-dihydro-4-oxo-3-chinolincarbonsäure,
- (±)-8-Chloro-1-cyclopropyl-6-fluoro-7-(trans-2-methyl-3-amino-1-azetidinyl)-1,4-dihydro-4-oxo-3-chinolincarbonsäure,
- 8-Chloro-1-cyclopropyl-6-fluoro-7-(3-methyl-3-amino-1-azetidinyl)-1,4-dihydro-4-oxo-3-chinolincarbonsäure,
- (±)-8-Chloro-1-(2,4-difluorophenyl)-6-fluoro-7-(trans-2-methyl-3-amino-1-azetidinyl)-1,4-dihydro-4-oxo-3-chinolincarbonsäure,
- 8-Chloro-1-(2,4-difluorophenyl)-6-fluoro-7-(3-methyl-3-amino-1-azetidinyl)-1,4-dihydro-4-oxo-3-chinolincarbonsäure,
- (±)-8-Chloro-1-(2-fluoroethyl)-6-fluoro-7-(trans-2-methyl-3-amino-1-azetidinyl)-1,4-dihydro-4-oxo-3-chinolincarbonsäure,

- 8-Chloro-1-(2-fluoroethyl)-6-fluoro-7-(3-methyl-3-amino-1-azetidinyl)-1,4-dihydro-4-oxo-3-chinolincarbonsäure,
- (±)-8-Chloro-6-fluoro-1-(4-fluorophenyl)-7-(trans-2-methyl-3-amino-1-azetidinyl)-1,4-dihydro-4-oxo-3-chinolincarbonsäure,
- 8-Chloro-6-fluoro-1-(4-fluorophenyl)-7-(3-methyl-3-amino-1-azetidinyl)-1,4-dihydro-4-oxo-3-chinolincarbonsäure,
- (-)-1-(2,4-Difluorophenyl)-6,8-difluoro-7-(3-(R)-amino-2-(S)-methyl-1-azetidinyl)-1,4-dihydro-4-oxo-3-chinolincarbonsäure.

**3.** Verbindungen der allgemeinen Formel (I) nach Anspruch 1, ausgewählt aus der folgenden Gruppe:
- 1-Cyclopropyl-6-fluoro-7-(3-amino-3-methyl-1-azetidinyl)-1,4-dihydro-4-oxo-1,8-naphtyridin-3-carbonsäure,
- 1-Cyclopropyl-6-fluoro-7-(trans-3-amino-2-methyl-1-azetidinyl)-1,4-dihydro-4-oxo-1,8-naphtyridin-3-carbonsäure,
- (-)-1-Cyclopropyl-6-fluoro-7-(3-(S)-amino-2-(R)-methyl-1-azetidinyl)-1,4-dihydro-4-oxo-1,8-naphtyridin-3-carbonsäure,
- (+)-1-Cyclopropyl-6-fluoro-7-(3-(R)-amino-2-(S)-methyl-1-azetidinyl)-1,4-dihydro-4-oxo-1,8-naphtyridin-3-carbonsäure,
- 1-Cyclopropyl-6-fluoro-7-(3-methyl-3-methylamino-1-azetidinyl)-1,4-dihydro-4-oxo-1,8-naphtyridin-3-carbonsäure,
- 6-Fluoro-7-(trans-2-methyl-3-amino-1-azetidinyl)-1-cyclopropyl-1,4-dihydro-4-oxo-1,8-naphtyridin-3-carbonsäure,
- (±)-1-(2,4-Difluorophenyl)-6-fluoro-7-(trans-2-methyl-3-amino-1-azetidinyl)-1,4-dihydro-4-oxo-1,8-naphtyridin-3-carbonsäure,
- (±)-6-Fluoro-1-(2-fluoroethyl)-7-(trans-2-methyl-3-amino-1-azetidinyl)-1,4-dihydro-4-oxo-1,8-naphtyridin-3-carbonsäure,
- 6-Fluoro-1-(2-fluoroethyl)-7-(3-methyl-3-amino-1-azetidinyl)-1,4-dihydro-4-oxo-1,8-naphtyridin-3-carbonsäure,
- (±)-6-Fluoro-1-(4-fluoroethyl)-7-(trans-2-methyl-3-amino-1-azetidinyl)-1,4-dihydro-4-oxo-1,8-naphtyridin-3-carbonsäure,
- 6-Fluoro-1-(4-fluorophenyl)-7-(3-methyl-3-amino-1-azetidinyl)-1,4-dihydro-4-oxo-1,8-naphtyridin-3-carbonsäure.

**4.** Verbindungen der allgemeinen Formel (I) nach Anspruch 1, ausgewählt aus der folgenden Gruppe:
- (-)-(3S)-10-[3-(S)-Amino-2-(R)-methyl-1-azetidinyl]-9-fluoro-2,3-dihydro-3-methyl-7-oxo-7H-pyrido-[1,2,3-de][1,4]benzoxazin-6-carbonsäure.
- (+)-(3R)-10-[3-(R)-Amino-2-(S)-methyl-1-azetidinyl]-9-fluoro-2,3-dihydro-3-methyl-7-oxo-7H-pyrido [1,2,3-de][1,4]benzoxazin-6-carbonsäure.
- (-)-(3S)-10-[3-(R)-Amino-2-(S)-methyl-1-azetidinyl]-9-fluoro-2,3-dihydro-3-methyl-7-oxo-7H-pyrido-[1,2,3-de][1,4]benzoxazin-6-carbonsäure.
- (+)-(3R)-10-[3-(S)-Amino-2-(R)-methyl-1-azetidinyl]-9-fluoro-2,3-dihydro-3-methyl-7-oxo-7H-pyrido[1,2,3-de][1,4]benzoxazin-6-carbonsäure.

**5.** Verfahren zur Herstellung der Verbindungen nach einem der Ansprüche 1 is 4, dadurch gekennzeichnet, daß man eine Verbindung der allgemeinen Formel (II)

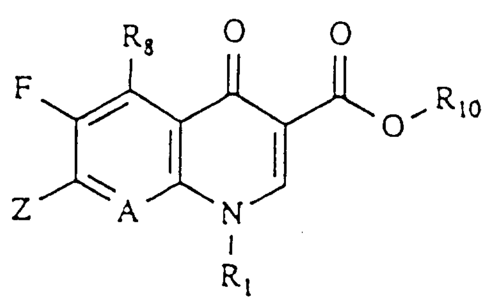

II

in der A, $R_1$, $R_8$ und $R_{10}$ die oben angegebenen Bedeutungen haben und Z ein Halogenatom, vorzugsweise ein Chlor- oder Fluoratom, darstellt,
mit einem Azetidin der allgemeinen Formel (III) umsetzt

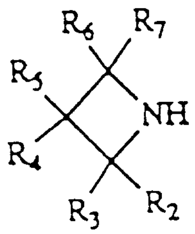

III

in der $R_2$, $R_3$, $R_4$, $R_5$, $R_6$ und $R_7$ die oben angegebenen Bedeutungen haben.

**6.** Die Derivate der allgemeinen Formel (I) und ihre therapeutisch akzeptablen Salze nach den Ansprüchen 1 bis 4 als Arzneimittel, insbesondere als Arzneimittel für die Behandlung bestimmter Infektionserkrankungen.

**7.** Pharmazeutische Zusammensetzungen, dadurch gekennzeichnet, daß sie außer einem pharmazeutisch akzeptablen Träger mindestens ein Derivat der allgemeinen Formel (I) oder eines seiner physiologisch akzeptablen Salze nach einem der Ansprüche 1 bis 4 enthalten.

**8.** Verwendung der Derivate der allgemeinen Formel (I) und ihrer physiologisch akzeptablen Salze nach einem der Ansprüche 1 bis 4 zur Herstellung von Arzneimitteln für die Behandlung von Mikroben-Infektionen.